# EUROPEAN PATENT APPLICATION

(11) **EP 2 722 400 A2**
(43) Date of publication of application: **23.04.2014**
(21) Application number: 13181855.1
(22) Date of filing: 22.10.2009
(51) Int. Cl.: C12Q 1/68, G01N 33/574

(54) **Methods of using biomarkers**

(30) Priority: 29.10.2008 US 109331 P
(62) Divisional of application: 09825206.7
(71) Applicant: William Beaumont Hospital, Royal Oak, MI 48073 (US)
(72) Inventor: Akervall, Jan, Ann Arbor, MI 48105 (US)
(74) Representative: Cohausz & Florack

(57) **Abstract**

The present invention provides methods and compositions for predicting patient responses to cancer treatment using the biomarkers: YAP-1, bcl-2, VEGF-c, c-met, and claudin-4.

## Description

### RELATED APPLICATION

This application claims the benefit of U.S. Provisional Application No. 61/109,331, filed October 29, 2008, the entire contents of which are incorporated herein by reference.

### BACKGROUND

Primary surgical therapy for oropharyngeal and laryngeal SCHNN (squamous cell carcinoma of the head and neck) has given way to definitive radiotherapy or concurrent chemoradiotherapy as the sole modality or with chemotherapy in favor of organ preservation. To support this, randomized trials have proven the benefit of using radiation therapy (RT) for organ preservation in patients with squamous cell carcinoma of the head and neck and a reported local control benefit as well as improvement in survival with the delivery of chemotherapy concurrently with radiotherapy (CRT). Now, with the advent of recent imaging modalities such as PET/CT (positron emission tomography/ computed tomography) there are fewer planned neck dissections in favor of observing the neck in patients with initial nodal positive disease that has a clinical response to CRT.

The above mentioned studies have provided the basis for current treatment decision which are primarily based on the patient's tumor-nodal-metastases (TNM) staging. However, patients with the same TNM stages have heterogeneous responses to therapy. Because these patients are often receiving RT and CRT as the sole therapy it becomes important to determine how each patient could respond to their respective therapy, and to separate those who are at high risk for local recurrence.

Due to the heterogeneous nature of tumors, it is less likely that any one specific marker will have prognostic or predictive value. Thus, there is a need in the art to identify biomarkers for use in assessing pre-treatment biopsies to predict the clinical response to RT and CRT in patients with head and neck cancer.

### SUMMARY

In one aspect, the present invention provides for methods for predicting the response to chemoradiotherapy treatment in a patient suffering from a head or neck cancer to comprising: measuring in a biological sample from said patient the protein or mRNA levels of (i) YAP-1; or (ii) at least one biomarker selected from (b) and at least one biomarker from (c): (b) bcl-2, and VEGF-c, (c) c-met, and claudin-4; or (iii) a combination of (i) and (ii). In certain embodiments, the mRNA levels are determined by measuring the levels of one or more nucleic acid sequences selected from the group consisting of: SEQ ID NO: 1 (YAP-1); SEQ ID NO: 2 (bcl-2);SEQ ID NO: 3 (VEGF-c); SEQ ID NO: 4 (c-met); and SEQ ID NO: 5 (claudin-4). In other embodiments, the protein levels are determined by measuring the levels of one or more amino acid sequences selected from the group consisting of: SEQ ID NO: 6 (YAP-1); SEQ ID NO: 7 (bcl-2); SEQ ID NO: 8 (VEGF-c); SEQ ID NO: 9 (c-met); and SEQ ID NO: 10 (claudin-4). In particular embodiments, the head or neck cancer is squamous cell carcinoma of the head and neck. In other embodiments, the cancer is oropharyngeal or laryngeal squamous cell carcinoma.

Further objects, features and advantages of this invention will become readily apparent to persons skilled in the art after a review of the following description, with reference to the drawings and claims that are appended to and form a part of this specification.

### BRIEF DESCRIPTION OF THE DRAWINGS

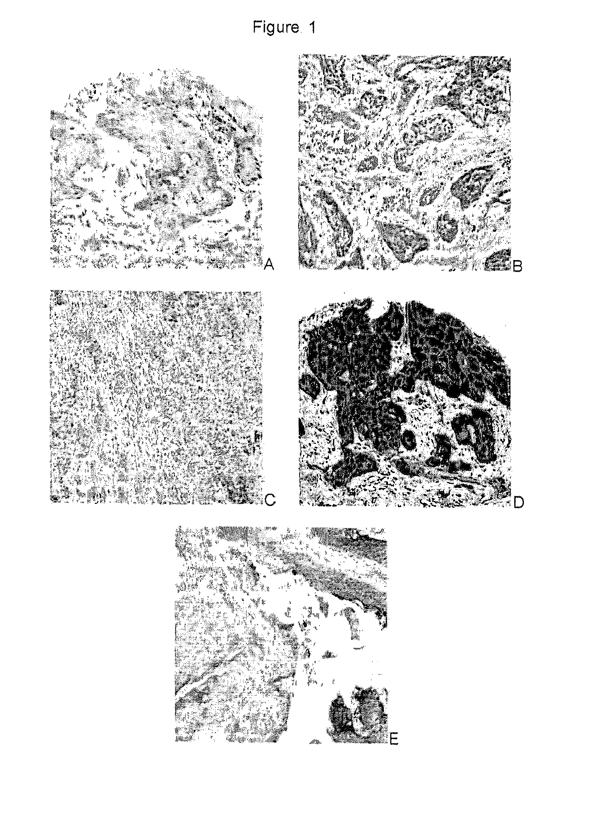

FIG. 1 depicts immunohistochemical staining of YAP-1 (A), bcl-2 (B), VEGF-c (C), c-met (D), claudin-4 (E) in biopsy specimens of patients with head and neck squamous cell carcinoma;

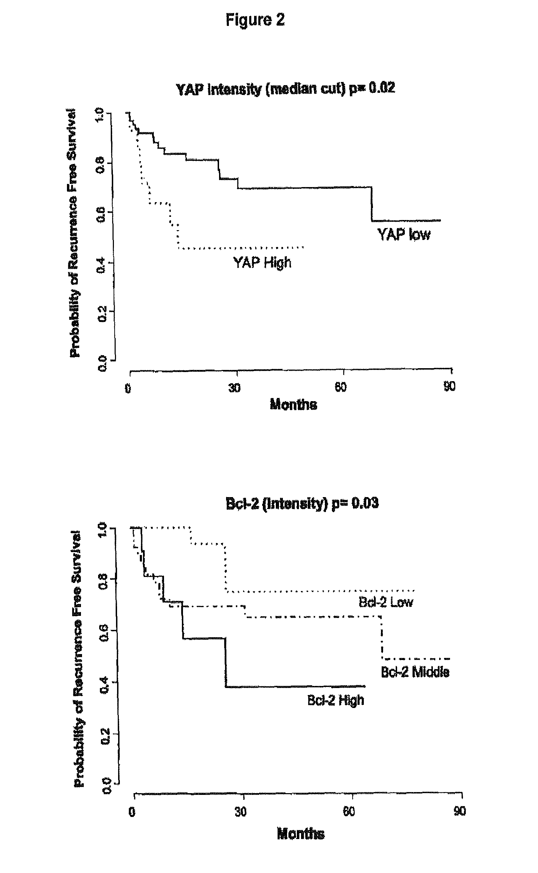

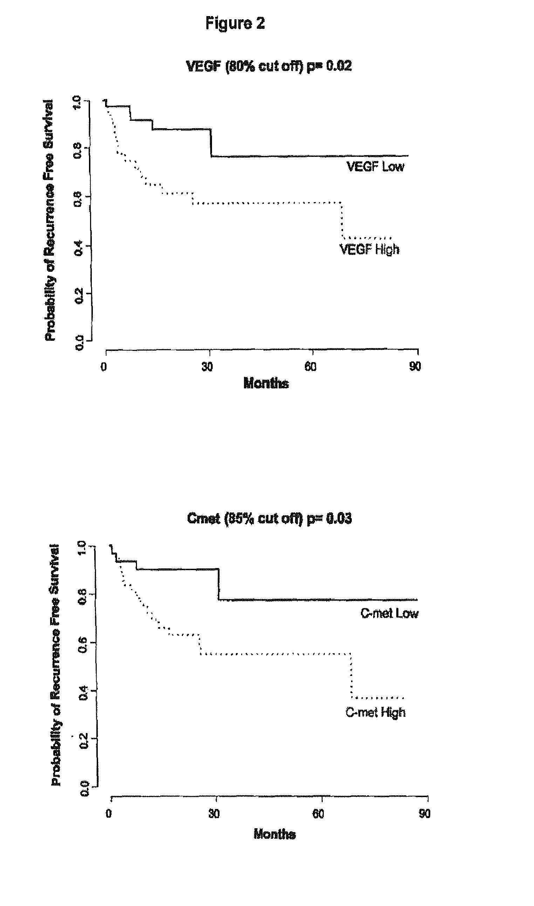

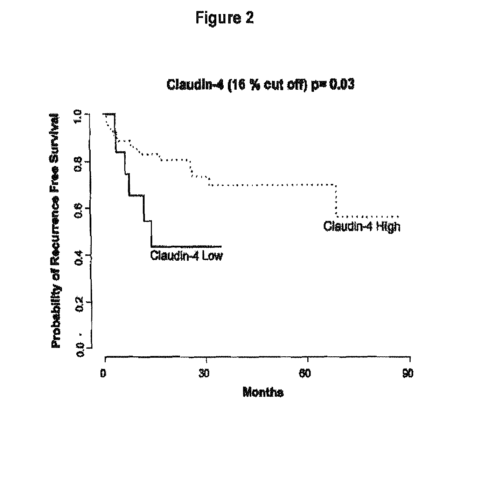

FIG. 2 depicts a Kaplan-Meier curve of the probability of recurrence free survival versus months for patients expressing high and low YAP-1, bcl-2, VEGF-c, c-met, and claudin-4;

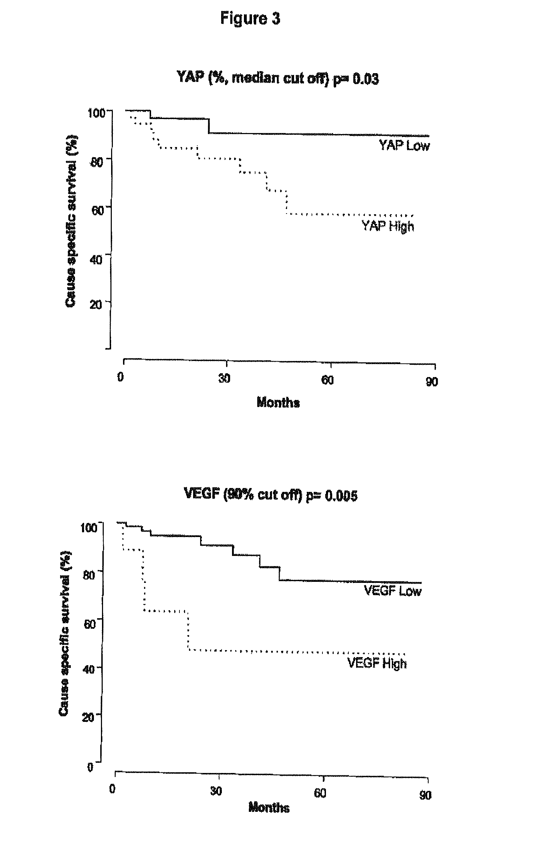

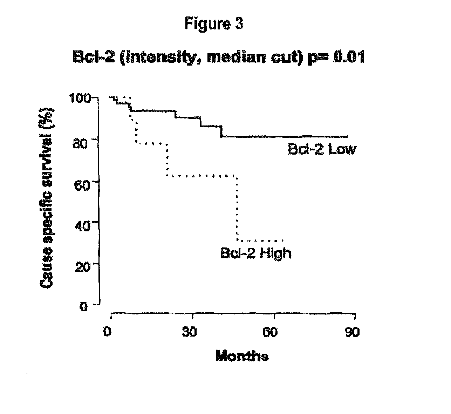

FIG. 3 depicts a Kaplan-Meier curve of the cause specific survival (%) versus months for patients expressing high and low levels of YAP-1, bcl-2, and VEGF-c.

### DETAILED DESCRIPTION

The present invention provides methods and compositions for the use of biomarkers to predict the response to chemoradiotherapy of a head and neck cancer patient. In particular the present invention provides for methods for predicting the response to chemoradiotherapy treatment in a patient suffering from a head or neck cancer to comprising: measuring in a biological sample from said patient the protein or mRNA levels of (i) YAP-1; or (ii) at least one biomarker selected from (b) and at least one biomarker from (c): (b) bcl-2, and VEGF-c, (c) c-met, and claudin-4; or (iii) a combination of (i) and (ii).

In certain embodiments, the methods involve measuring in a biological sample from a patient the nucleic acid levels of one or more of YAP-1 (Yes-associated protein 65 kDa), bcl-2 (B-cell CLL/lymphoma 2), VEGF-c (vascular endothelial growth factor C), c-met, or claudin-4. Examples of nucleic acids associated with each of YAP-1, bcl-2, VEGF-c, c-met, or claudin-4 are in Table 1:

**Table 1**

| **Biomarker** | **SEQ ID NO:** | **GenBank Accession No.** |
|---|---|---|
| YAP-1 | SEQ ID NO: 1 | NM_006106 |
| bcl-2 | SEQ ID NO: 2 | M14745 |
| VEGF-c | SEQ ID NO: 3 | BC035212 |
| c-met | SEQ ID NO: 4 | NM_000245 |
| claudin-4 | SEQ ID NO: 5 | NM_001305 |

Examples of expressed sequence tag nucleic acid sequences that are associated with each of YAP-1, c-met, or claudin-4 are in Table 2:

**Table 2**

| **Biomarker** | **SEQ ID NO:** | **GenBank Accession No.** |
|---|---|---|
| YAP-1 | SEQ ID NO: 11 | AA708798 |
| c-met | SEQ ID NO: 12 | AA191433 |
| claudin-4 | SEQ ID NO: 13 | AA430665 |

In certain embodiments, the methods involve measuring in a biological sample from a patient the protein levels of one or more of YAP-1, bcl-2, VEGF-c, c-met, or claudin-4. Examples of amino acid sequences associated with each of YAP-1, bcl-2, VEGF-c, c-met, or claudin-4 are in Table 3:

**Table 3**

| **Biomarker** | **SEQ ID NO:** | **GenBank Accession No.** |
|---|---|---|
| YAP-1 | SEQ ID NO: 6 | NP_006097 |
| bcl-2 | SEQ ID NO: 7 | AAA35591 |
| VEGF-c | SEQ ID NO: 8 | AAH35212 |
| c-met | SEQ ID NO: 9 | NP_000236 |
| claudin-4 | SEQ ID NO: 10 | NP_001296 |

To examine the levels of mRNA or protein expression of one or more biomarkers, a biological sample of a head or neck cancer patient is typically assayed. A "biological sample" includes a sample from a tumor, a cancerous tissue, a pre-cancerous tissue, a biopsy, blood, serum, saliva, or a tissue, etc. obtained from a patient suffering from a head or neck cancer or who has yet to be diagnosed with a head or neck cancer.

The biological sample is then typically assayed from the presence of one or more expression products of a biomarker gene such as mRNA, cDNA, cRNA, protein, etc.

In one embodiment, a sample comprising RNA from a biological sample is used directly to measure the mRNA levels of a biomarker. In one particular embodiment, RNA is obtained from a biological sample. The RNA is then transformed into cDNA (complementary DNA) copy using methods known in the art. In particular embodiments, the cDNA is labeled with a fluorescent label or other detectable label. The cDNA is then hybridized to a substrate containing a one or more probes of interest. A probe of interest typically hybridizes under stringent hybridization conditions to the DNA sequence of interest. In certain embodiments, one or more nucleic acid probes are capable of hybridizing to the sequences of interest (e.g., any of SEQ ID NOS: 1-5, 11-13, or fragments thereof (e.g., fragments at least 15 nucleotides in length)) under the hybridization conditions of 6X SSC (0.9 M NaCl, 0.09 M sodium citrate, pH 7.4) at 65°C. The probes may comprise nucleic acids. An example of a nucleic acid is DNA. The term "nucleic acid" refers to deoxyribonucleotides or ribonucleotides and polymers thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, and peptide-nucleic acids (PNAs).

In certain cases, the probes will be from about 15 to about 50 base pairs in length. The amount of cDNA hybridization can be measured by assaying for the presence of the detectable label, such as a fluorophore. The amount of the hybridization signal can be used to generate a qualitative or quantitative measurement of the level of a nucleic acid of interest in sample.

The term "detectable label" refers to a moiety that is attached through covalent or non-covalent means to an entity being measured or a probe. A "detectable label" can be a radioactive moiety, a fluorescent moiety, a chemiluminescent moiety, etc. The term "fluorescent label" refers to label that accepts radiant energy of one wavelength and emits radiant energy of a second wavelength. The presence of a detectable label may be assayed using methods known in the art that are appropriate to detect a particular label, such as spectrophotometric means (e.g., a spectrophotometer), radiometric means (e.g., scintillation counter), fluorometer, luminometer, etc.

Included within the scope of the invention are DNA microarrays containing a plurality of sequences that hybridize under stringent hybridization conditions to one or more biomarker gene sequences. An example of a substrate containing one or more probes of interest is a plurality of DNA probes that are affixed to a substrate. In certain embodiments, the substrate may comprise one or more materials such as gel, nitrocellulose, nylon, quartz, glass, metal, silica based materials, silica, resins, polymers, etc., or combinations thereof. Typically, the DNA probes comprise about 10-50 bp of contiguous DNA. In certain embodiments, the DNA probes are from about 20 to about 50 bp of contiguous DNA. In certain embodiments, the present invention relates to kits which comprise a microarray of one or more sequences capable of stringently hybridizing to any of SEQ ID NOS: 1-5, 11-13 or the complementary strand of any of SEQ ID NOS: 1-5, 11-13 and its directions for its use. The kit may comprise a container which comprises one or more microarrays and directions for their use.

The biological sample may also be analyzed for mRNA of one or more biomarkers using methods that can detect nucleic acids including, but not limited to, PCR (polymerase chain reaction); RT-PCR (reverse transcriptase-polymerase chain reaction); quantitative PCR, etc.

In certain embodiments, the levels of biomarker protein are measured by detecting the protein expression products of the genes or DNA sequences (e.g., any of SEQ ID NOS: 6-10). The levels of protein products may be measured using methods known in the art including the use of antibodies which specifically bind to a particular protein. These antibodies, including polyclonal or monoclonal antibodies, may be produced using methods that are known in the art. These antibodies may also be coupled to a solid substrate to form an antibody chip or antibody microarray. Antibody or protein microarrays may be made using methods that are known in the art. In addition, immunoassays, including immunohistochemistry, may be employed. In certain embodiments, the present invention relates to kits which comprise reagents (such as antibodies) capable of specifically binding to any of SEQ ID NOS: 6-10 and its directions for its use. The kit may comprise a container which comprises one or more reagents and directions for their use. Furthermore, mass spectrometry may be used to detect proteins or fragments thereof, and may be used in combination with other techniques such as HPLC.

The treatment of head and neck cancer in certain embodiments, involves measuring the levels of mRNA or protein of one or more biomarkers selected from the group consisting of YAP-1, bcl-2, VEGF-c, c-met, and claudin-4. The method of treatment typically further comprises administering a therapeutically effective amount of one or more cancer treatment agents selected from the group consisting of: cancer chemotherapeutic agents and radiation. The treatment of cancer may also comprise surgery or surgical procedures. The term "administering" refers to the method of contacting a compound with a subject. Modes of "administering" may include but are not limited to, methods that involve contacting the cancer chemotherapeutic agents intravenously, intraperitoneally, intranasally, transdermally, topically, via implantation, subcutaneously, parentally, intramuscularly, orally, systemically, and via adsorption. The term "treatment" includes the acute or prophylactic diminishment or alleviation of at least one symptom or characteristic associated or caused by the cancer being treated. For example, treatment can include diminishment of several symptoms of a cancer or complete eradication of a cancer. The phrase "therapeutically effective amount" means an amount of a cancer chemotherapeutic agent, or a pharmaceutically acceptable salt thereof, that is sufficient to inhibit, halt, or allow an improvement in the cancer being treated when administered alone or in conjunction with another pharmaceutical agent or treatment in a particular subject or subject population. For example in a human a therapeutically effective amount can be determined experimentally in a clinical setting, for the particular disease and subject being treated. It should be appreciated that determination of proper dosage forms, dosage amounts and routes of administration is within the level of ordinary skill in the pharmaceutical and medical arts.

It is within the purview of the skill medical practitioner to select an appropriate therapeutic regimen. Therapeutic regimens may be comprised of the use of cancer chemotherapeutic agents and/or radiation. Chemoradiotherapy is the use of both radiation and chemotherapy to treat a patient suffering from a cancer. The radiation and chemotherapy do not have to occur simultaneously and can be separated in time, for example by hours, days, or months, etc. A cancer chemotherapeutic agent is a chemical or biological agent (e.g., antibody, protein, RNA, DNA, etc.) that retards, slows, or stops the growth of cancer or is approved to treat a cancer by the U.S. Food and Drug Administration. Examples of head and neck cancer chemotherapeutic agents include, but are not limited to cisplatin, cetuximab, docetaxel, and erlotinib. In particular cases, the chemoradiotherapy comprises administering cisplatin and 5-fluorouracil. Another example of a cancer treatment agent is radiation. In certain embodiments, the cancer is a head or neck cancer. Examples of head or neck cancer include, but are not limited to: squamous cell carcinoma of the head and neck. Further examples of head and neck cancers include oropharyngeal and laryngeal squamous cell carcinoma.

### EXAMPLES

**Purpose:** To correlate expression of VEGF-c, bcl-2, claudin-4, c-met, and YAP-1 in pre-treatment biopsies with clinical outcomes in patients with squamous carcinoma of the head and neck (SCCHN).

**Methods:** From December 1995 to November 2004, 86 patients with clinical stage II-IVa SCCHN who underwent radiotherapy (RT) alone or concurrent cisplatinum based chemoradiotherapy (CRT) were selected. Immunohistochemical staining (IHC) for VEGF-c, bcl-2, claudin-4, YAP-1 and c-met proteins was performed on pretreatment biopsy specimens obtained from all 86 patients. Staining was graded according to intensity and percentage of cells positive by two independent observers.

**Results:** The median follow-up was 33.8 months. Twelve patients experienced an IR (incomplete response) and 11 patients recurred at a median time of 12.6 months. Of the recurrences, 7 patients were found to have a LRR (locoregional recurrence) and 4 patients were found to have distant metastases. Cause specific survival (CSS) and recurrence free survival (RFS) at 2 years was 85% and 90% and at 3 years was 81% and 84%, respectively. Biomarkers predictive for IR were increased VEGF-c (p=0.02), YAP-1(p<0.01), claudin-4 (p<0.01), c-met (p<0.01) and bcl-2 (p=0.02). Biomarkers predictive of RFS were YAP-1 (p=0.01) and bcl-2 (p<0.01). Biomarkers predictive of CSS were YAP-1(p=0.04), VEGF-c (p=0.03), and claudin-4 (p=0.03).

**Conclusion:** All biomarkers were predictive of IR; in addition claudin-4 and VEGF-c predicted for CSS and bcl-2 for RFS. YAP-1 was the universal marker in predicting for all endpoints. Analyzing individual genetic profiles in the clinical setting using the above markers may allow for tailored patient-specific therapy to improve outcomes.

### MATERIALS AND METHODS

### Patients

One hundred and thirty one patients consecutively treated with primary RT or CRT for squamous cell carcinoma of the oropharynx and larynx from May 1995-July 2004 at William Beaumont Hospital were identified for this Human Investigation Committee approved study. Forty-five patients were excluded from the analysis due to previous history of carcinoma excluding non-melanomatous skin cancer, treatment received at an outside institution, unavailable treatment record or lack of tissue available for analysis. From December 1995 to November 2004, 86 patients with clinical stage II-IVa oropharyngeal (n=30) and stage I-IVa laryngeal (n=56) SCCHN who underwent treatment with either RT alone (n=47) or CRT (n=39) were selected for analysis.

### Treatment

The primary site was treated with 6 mV photons. For early stage larynx patients, two-dimensional radiotherapy planning utilizing a 5x5 or 6x6 field box was used. Prior to 2004, most of the remaining patients were treated with three-dimensional conformal radiotherapy; thereafter, intensity modulated radiotherapy was used to maximally spare normal tissues. Fifteen patients received treatment twice daily, five days per week, with 120 cGy (centigray) per fraction. Seventy-one patients received treatment once daily, five days per week, with fraction dose ranging from 180-225 cGy per fraction. Median total dose to the primary site was 7000 cGy, (ranged from 2400 cGy -8160 cGy), and the median overall treatment time was 50 days (range, 16 to 69 days). One patient received 2400 cGy in a hypofractionated regimen secondary to difficulty with treatment compliance. All CRT patients received platinum based chemotherapy delivered concurrently with the radiotherapy. Treatment response was evaluated during and after treatment using nasopharyngoscopy, computed tomography, and/or biopsies. Any locoregional recurrence within 6 months after completion of radiation therapy was considered an incomplete response defined as either a partial response or no response.

### Tumor samples

Tumor samples were collected with the approval of the institutional human investigational committee from the eighty-six patients as described above. All of the tumors were fixed in formalin and embedded in paraffin wax. A head and neck pathologist reviewed the histopathology of each case by light microscopy of the hematoxylin and eosin stained sections and identified the appropriate regions of tissue for the immunohistochemistry to be performed.

### Tissue array

One to four 1.5 mm punch biopsies were taken from the paraffin embedded specimens after microscopic identification of representative tumor containing areas in hematoxylin/eosin (H&E) stained sections. The punch biopsies were then mounted on new paraffin blocks, yielding up to 100 specimens in each individual block. Five micrometer sections were then cut in a regular fashion for immunohistochemical analysis.

### Immunohistochemistry

Immunohistochemical staining (IHC) for VEGF, bcl-2, claudin-4, YAP-1 and c-met was performed on pretreatment biopsy specimens obtained from all 86 patients. Tissue array technique was used to analyze areas of interest in each specimen. These areas were marked together with a pathologist. Hematoxylin/Eosin (H&E) was used to stain specific sections. To detect the five proteins, immunohistochemistry was completed using the Discovery XT System (Ventana, Tucson, AZ) at the following dilutions: bcl-2 (1:200), c-met (1:50), claudin-4 (1:50), VEGF-c (1:100) and YAP-1 (1:25). Antigen retrieval was performed using citrate buffer at pH 6 at 95°C for 25 minutes. For quenching of endogenous peroxidases, sections were blocked with normal horse serum 2.5% (R.T.U. Vectastain Kit, Vector Laboratories, Burlingame, CA) for 20 minutes. A secondary antibody was added (R.T.U. Biotinylated Universal antibody Anti-rabbit/mouse IgG, Vectastain ABC-kit, Vector Laboratories, Burlingame, CA) for 30 minutes. After rinsing in PBS, specimens were incubated in ABC-reagent (Vectastain ABC-kit, Vector Laboratories, Burlingame, CA) for 30 minutes, and counterstained with hematoxylin.

The slides were then scored by two independent observers; staining was graded according to intensity and percentage of cells positive. If there was a discrepancy between the two independent observations, a third independent observer scored the slides and the results were averaged.

### Statisfical Analysis

Incomplete response was defined as either gross or microscopic persistent viable tumor after treatment or recurrence of tumor within six month after completion of treatment. Recurrence-free survival was defined as the time from radiotherapy completion to the first relapse (after six months from completion of treatment), death, or last follow-up, whichever occurred first. Cause specific survival was defined as the time from radiotherapy completion to death due to disease or last follow-up. Cumulative survival probabilities were plotted using Kaplan-Meier curves and compared with log-rank test. All tests were declared statistically significant if the calculated p value was ≤ 0.05. The chi-squared test was used to detect any correlation between biomarkers and clinical treatment factors. The statistical analysis was performed with Version 5.0 of the SAS statistical software package (SAS Institute Inc, Cary, NC) and (R version 2.6.1).

### RESULTS

### Patient/tumor characteristics

Median follow up was 29 months. The clinicopathologic characteristics are summarized in Table 4. As can be seen, the median age was 63 years (range, 40-95 years). Sixty-nine (80%) of 86 patients were male. Thirty-one patients had oropharyngeal primaries and 55 had laryngeal primaries. Of the laryngeal SCCHN, 41 patients had T1-T2 lesions, 14 patients had T3-T4 lesions, and 10 had locoregional lymph node involvement. Of the oropharyngeal SCCHN, 19 patients had T1-T2 lesions, 12 patients had T3-T4 lesions, and 22 patients had lymph node involvement.

**Table 4**

| **Patient Characteristics** | | |
|---|---|---|
| **Factor** | | ***n* (%)** |
| **Sex** | | |
| | Male | 69 (80%) |
| | Female | 17 (20%) |

| **Subsite** | | |
|---|---|---|
| | Oropharynx | 31 (36%) |
| | Larynx | 55 (64%) |

| **T stage** | | |
|---|---|---|
| | T1-T2 | 60 (70%) |
| | T3-T4 | 26 (30%) |

| **Nodal Status** | | |
|---|---|---|
| | N0 | 54 (63%) |
| | N+ | 32 (37%) |

| **AJCC Stage** | | |
|---|---|---|
| | I | 20 (23%) |
| | II | 18 (21%) |
| | III | 20 (23%) |
| | IV | 28 (33%) |

| **Chemotherapy** | | |
|---|---|---|
| | Yes | 39 (45%) |
| | No | 47 (55%) |

### Clinical Outcome

Patients were evaluated during and after treatment for response via clinical exam, nasopharyngoscopy, radiographic study, or biopsy. Twelve patients (7 larynx, 5 oropharynx) experienced an incomplete response; clinical and treatment characteristics for these patients are summarized in Table 5. No patient with an incomplete response or recurrence received less than 6600 cGy. The only significant clinical factor in the prediction of an incomplete response was having a radiation course time of greater than 50 days (p=0.04). There was an increased chance of experiencing distant failure with an incomplete response to therapy (p<0.01).

**Table 5**

| **Clinical and treatment characteristic of patients with incomplete response** | | | | |
|---|---|---|---|---|
| Patient | Site | Overall Stage | T Stage | N Stage |
| 1 | Larynx | 1 | 1a | 0 |
| 2 | Larynx | 1 | 1a | 0 |
| 3 | Larynx | 2 | 2 | 0 |
| 4 | Oropharynx | 2 | 2 | 0 |
| 5 | Oropharynx | 3 | 3 | 0 |
| 6 | Larynx | 3 | 3 | 0 |
| 7 | Larynx | 3 | 3 | 0 |
| 8 | Larynx | 3 | 3 | 0 |
| 9 | Larynx | 3 | 2 | 1 |
| 10 | Oropharynx | 4a | 1 | 2 |
| 11 | Oropharynx | 4a | 4 | 2a |
| 12 | Oropharynx | 4b | 4 | 3 |

Eleven patients (8 larynx, 3 oropharynx) were noted to have recurrence with a median time to recurrence of 12.5 months (range, 7.6 - 67.8 months). Of the recurrences, 7 patients were found to have a locoregional recurrence (median, 10.8 months) and 4 patients were found to have distant metastases (median, 25.2 months).

Recurrence free survival (RFS) and cause specific survival (CSS) at 2 years was 90% and 85% and at 3 years was 84% and 81 %, respectively. Treatment variables analyzed including age, gender, primary site, clinical stage, primary treatment, radiation dose, and elapsed days during the radiation course. Univariate analysis revealed older age (p=0.04) and primary treatment (RT vs. CRT) (p=0.03) to predict for RFS. Primary treatment course was then analyzed in Stage < =2 (n=38) vs. Stage >2 (n=48) and node positive patients (n=54) vs. node negative patients (n=32). Chemoradiation has significant effect on Stage 3 and 4 patient group and Node positive patient groups, as expected. Older age when analyzed as a continuous variable (p= 0.04) and age greater or less than 62 (p=0.03) as well as higher clinical T stage (0.03) predicted for CSS on UVA (univariate analysis).

### Predictive Biomarkers

### Biomarkers Predictive of Incomplete Response

Biomarkers predictive for incomplete response were increased VEGF-c optimal cut of 78.6% (p=0.02), YAP-1 intensity grading (p<0.01), YAP-1 intensity grading as a continuous variable (p<0.01), claudin-4 optimal cut of 85.1% (p<0.01), c-met intensity grading (p<0.01) and bcl-2 intensity grading (p=0.02).

### Biomarkers Predictive of Recurrence Free Survival

Biomarkers predictive of RFS were YAP-1 optimal cut of 37.8% (p=0.01), YAP-1 intensity grading (p=0.03), and bcl-2 optimal cut of 10% (p<0.01).

### Biomarkers Predictive of Cause Specific Survival

Biomarkers predictive of CSS were YAP-1 median intensity grading (p=0.04), YAP-1 optimal cut of 81.8% (p=0.02), VEGF-c optimal cut of 78.6% (p=0.03), and claudin-4 optimal cut of 85.1% (p=0.03).

Significant biomarkers were tested against significant clinical factors for each end point to test the independent prognostic significance of the biomarker. YAP-1 and bcl-2 were found to be independent of age at diagnosis in predicting for RFS, YAP-1 and VEGF-c were independent of age at diagnosis for CSS and VEGF-c was independent of Clinical T stage as a continuous variable for CSS. All biomarkers were independent of days of radiation in predicting for incomplete response. Interestingly, a log rank score was evaluated for both CSS and RFS and revealed a combination of clinical stage, claudin-4, YAP-1 and age to be very significant for CSS (p<0.01) and age, YAP-1, claudin-4 and primary treatment to be significant for RFS (p=0.02).

When the biomarkers YAP-1, bcl-2, VEGF-c, c-met, and claudin-4 were analyzed with regards to grade of positive cells and percentage of positive cells, the combination of the two, i.e. grade multiplied by percentage, had a synergistic effect. The combination of grade and percentage according to this formula improved the significance of the biomarkers when analyzed for response to therapy and prognosis. Furthermore, the combination of c-met and YAP-1 was a more significant predictor of response to radiation based therapy in the entire population (n=86), with 90% correct prediction of outcome of therapy, as compared to when these 2 markers were analyzed in subgroups consistent of either patients treated with radiation alone or in patients treated with chemoradiation.

### DISCUSSION

As primary treatment of SCCHN has shifted towards the use of radiotherapy or chemoradiotherapy, predicting tumor response to both modalities is useful in tailoring patient specific therapy. Treatment response to therapy was found to be independent of TNM stage in this study and patients with an incomplete response to their primary therapy (RT or CRT) were more likely to experience distant metastasis and have lower CSS than those with complete response. Therapy can be guided by evaluating individual tumor biology by assessing established prognostic and predictive biomarkers; this approach is already in use in early stage breast cancer to determine the benefit of chemotherapy.

The Yes Associated Protein (YAP-1) was found to be the universal biomarker in this study. The overexpression of YAP-1 has been seen in ependymoma (Modena et al. (2006)), NSCLC (non-small cell lung cancer) (Saviozzi et al. 2006), and pancreatic cancer (Guo et al. (2006)).

A previous study has shown that genes for the tyrosine kinase Yes-associated protein (YAP65) were preferentially expressed in transformed and metastatic tumor cell lines (Dong et al. (1997)). Silencing of YAP-1 reduces histone acetylation on the p53, resulting in delayed or reduced apoptosis mediated by p73 (Strano et al. (2005)).

Previous work has found low c-met expression to be associated with cisplatin sensitivity, and during the previous study concluded patients with tumors having high expression of c-met may not be good candidates for concomitant chemoradiation (Akervall et al. (2004)). This has been validated as in this study, c-met was significant in predicting for poor response to radiotherapy or chemoradiotherapy. MET is a tyrosine kinase receptor involved in proliferation, mitogenesis, angiogenesis, and metastasis. Overexpression of Met has been reported in breast, ovarian, thyroid, pancreatic, brain, and gastrointestinal tumors, and c-met overexpression has been correlated with poor prognosis in nasopharyngeal carcinoma patients (Qian et al. (2002)). Overexpression of c-met has also been shown to be a predictor of local recurrence in oral tongue carcinoma (Endo et al. (2006)). Currently, c-met was not predictive in CSS. There was no prediction of c-met in RFS; this may due to the fact that patients who had an incomplete response were censored in the RFS analysis.

In our study, Claudin-4 was found to be significant in predicting RFS. Claudin-4 encodes tight junction proteins. Its high expression has been noted in patients with breast cancer, urothelial, and prostate cancer. Increased claudin-4 in HNSCC and NSCLC cell lines were found to be associated with increases sensitivity to gefitinib (Frederick et al. (2007)), but this is targeted therapy and the conclusion may not be valid in this study. In serous papillary carcinoma, overexpression of claudin-4 was associated with poorer DFS and OS, with overexpression seen in the aggressive phenotyopes (Konecny et al. (2008)). In urothelial and prostate cancer it was found to be associated with stage and metastases. Perhaps, claudin-4 is a surrogate marker for tumor spread, which is why it may have lead to worse RFS.

In this study, bcl-2 was significant in predicting recurrence free survival and cause specific survival. Historically, it was discovered to be an anti apoptotic oncogene, suppressing p53. Most recently, in a cohort of nasopharynx patients who underwent high dose radiotherapy, bcl-2 overexpression had worse 5 year DFS (Chen et al. (2008)); 21 patients with locally advanced SCCHN who were treated with chemoradiation were found to have unfavorable outcome and shorter RFS if they overexpressed bcl-2 (Mannarini et al. (2007)).

Tumor hypoxia has been associated with poor response to radiation therapy; in this study VEGF-c was found to have significance in predicting RFS and CSS. VEGF-c is an angiogenesis promoting oncogene; previously, high expression of VEGF-c has been linked to poor response to radiation therapy. Pathologic complete response was assessed in patients undergoing preoperative rectal cancer; carcinomas that were considered VEGF-c positive were noted to have a pathological incomplete response (Zlobec et al. (2008)). In 27 patients with Stage II-IV SCCHN, high expression of VEGF-c significantly reduced local control and survival (Martin et al. (2007)). High levels of VEGF-c may be a surrogate for tumor hypoxia; hypoxia leads to radioresistance.

There are some limitations to this study. The patients that were analyzed were a heterogeneous group with a mixture of different head and neck disease sites and difference TNM stages. Patterns of spread to locoregional lymph nodes differences amongst patients with glottic primaries versus oropharyngeal primaries. Also, as this study spanned a time period of 9 years, different radiotherapy techniques and chemotherapy regimens were used. However, a heterogeneous population similar to one identified in this study is more likely representative of the population encountered in the wide range of clinical practices throughout the country; therefore, it makes the use of the panel of markers more universally applicable. Also, the biases associated with all retrospective studies have to be considered.

### Conclusion

In this study, the prognostic and predictive abilities of five markers with potential importance for chemosensitivity or radiosensitivity (VEGF-c, bcl-2, claudin-4, c-met, and YAP-1) based on previous c-DNA microarray studies (published and unpublished) were evaluated. By assessing pre-treatment biopsies, YAP-1 was found to be a universal marker in predicting for RT/CRT response, CSS, and RFS. Claudin-4 and VEGF-c predict for both CSS and RFS, and bcl-2 predicts for RT/CRT response and RFS. By using the above tested biomarkers patients at high risk for local recurrence can be identified early and appropriate therapy can be delivered upfront to optimize cancer cure and decrease morbidity from necessary salvage therapy in addition to primary therapy.

### References

Akervall et al. "Genetic and expression profiles of squamous cell carcinoma of the head and neck correlate with cisplatin sensitivity and resistance in cell lines and patients." Clin Cancer Res. (2004) 10(24):8204-8213.
Chen et al. "Prognostic impact of bcl-2 expression on advanced nasopharyngeal carcinoma." Head Neck. (2008) 30(8):1052-1057
Dong, G. et al. "Genes differentially expressed with malignant transformation and metastatic tumor progression of murine squamous cell carcinoma." J Cell Biochem.Suppl 28-29 (1997): 90-100.
Endo et al. "Prognostic value of cell motility activation factors in patients with tongue squamous cell carcinoma." Hum Pathol. (2006) 37(8):1111-1116.
Frederick et al. "Epithelial to mesenchymal transition predicts gefitinib resistance in cell lines of head and neck squamous cell carcinoma and non-small cell lung carcinoma." Mol Cancer Ther. (2007) 6(6):1683-1691.
Guo, J. et al. "Yes-associated protein (YAP65) in relation to Smad7 expression in human pancreatic ductal adenocarcinoma." Int.J Mol.Med. 17.5 (2006): 761-67.
Konecny et al. "Claudin-3 and claudin-4 expression in serous papillary, clear-cell, and endometrioid endometrial cancer." Gynecol Oncol. (2008) 109(2):263-269.
Mannarini et al. "Markers of chemoradiation resistance in patients with locally advanced head and neck squamous cell carcinoma, treated by intra-arterial carboplatin and concurrent radiation." Acta Otorhinolaryngol Ital. (2007) 27(4):173-180
Martin et al. "Vascular endothelial growth factor expression predicts outcome after primary radiotherapy for head and neck squamous cell cancer." Clin Oncol (R Coll Radiol). (2007) 19(1):71-76
Modena, P. et al. "Identification of tumor-specific molecular signatures in intracranial ependymoma and association with clinical characteristics." J Clin.Oncol. 24.33 (2006): 5223-33.
Qian et al. "Met protein expression level correlates with survival in patients with late-stage nasopharyngeal carcinoma." Cancer Res. (2002)62(2):589-596
Saviozzi, S. et al. "Selection of suitable reference genes for accurate normalization of gene expression profile studies in non-small cell lung cancer." BMC.Cancer 6 (2006): 200.
Strano, S. et al. "The transcriptional coactivator Yes-associated protein drives p73 gene-target specificity in response to DNA Damage." Mol.Cell 18.4 (2005): 447-59.
Zlobec et al. "Combined analysis of VEGF and EGFR predicts complete tumour response in rectal cancer treated with preoperative radiotherapy." Br J Cancer. (2008) 98(2):450-456.

The present invention further comprises the aspects defined in the following clauses (which form part of the present description but are not considered as claims in accordance with decision J 15/88 of the Legal Board of Appeal of the European Patent Office):
1. A method for predicting the response to chemoradiotherapy treatment in a patient suffering from a head or neck cancer to comprising:
   measuring in a biological sample from said patient the protein or mRNA levels of:
      (i) YAP-1; or
      (ii) at least one biomarker selected from (b) and at least one biomarker from (c):
         (b) bcl-2, and VEGF-c,
         (c) c-met, and claudin-4; or
      (iii) a combination of (i) and (ii).
2. The method of clause 1, wherein the mRNA levels are determined by measuring the levels of one or more nucleic acid sequences selected from the group consisting of:
   SEQ ID NO: 1 (YAP-1);
   SEQ ID NO: 2 (bcl-2);
   SEQ ID NO: 3 (VEGF-c);
   SEQ ID NO: 4 (c-met); and
   SEQ ID NO: 5 (claudin-4).
3. The method of clause 1, wherein the protein levels are determined by measuring the levels of one or more amino acid sequences selected from the group consisting of:
   SEQ ID NO: 6 (YAP-1);
   SEQ ID NO: 7 (bcl-2);
   SEQ ID NO: 8 (VEGF-c);
   SEQ ID NO: 9 (c-met); and
   SEQ ID NO: 10 (claudin-4).
4. The method of clause 1, wherein said head or neck cancer is squamous cell carcinoma of the head and neck.
5. The method of clause 4, wherein said cancer is oropharyngeal or laryngeal squamous cell carcinoma.
6. The method of clause 1, wherein said biological sample is from a tumor, a cancerous tissue, a pre-cancerous tissue, a biopsy, blood, serum, saliva, or a tissue.
7. The method of clause 1, wherein said chemoradiotherapy comprises administering one or more agents selected from the group consisting of: cisplatin, cetuximab, docetaxel, and erlotinib.
8. The method of clause 7, wherein said chemoradiotherapy comprises administering cisplatin and 5-fluorouracil.
9. The method of clause 3, wherein the determination of the protein levels is carried out using immunohistochemistry, an immunoassay, a protein assay, mass spectrometry, immunofluorescence, or a combination thereof.
10. The method of clause 1, wherein the response to chemoradiotherapy treatment is whether the patient will have an incomplete response to the chemotherapy.

## Claims

1. A method for predicting the response to chemoradiotherapy treatment in a patient suffering from a head or neck cancer to comprising:
measuring in a biological sample from said patient the protein or mRNA levels of:
(i) YAP-1; or
(ii) At least one biomarker selected from (b) and at least one biomarker from (c):
(b) bcl-2, and VEGF-c;
(c) c-met, and claudin-4; or
(iii) a combination of (i) and (ii); or
measuring in a biological example from said patient the protein or mRNA levels of:
(i) YAP-1; and
(ii) c-met.

2. The method of claim 1, wherein the mRNA levels are determined by measuring the levels of one or more nucleic acid sequences selected from the group consisting of:
SEQ ID NO: 1 (YAP-1);
SEQ ID NO: 2 (bcl-2);
SEQ ID NO: 3 (VEGF-c);
SEQ ID NO: 4 (c-met); and
SEQ ID NO: 5 (claudin-4).

3. The method of claim 1, wherein the protein levels are determined by measuring the levels of one or more amino acid sequences selected from the group consisting of:
SEQ ID NO: 6 (YAP-1);
SEQ ID NO: 7 (bcl-2);
SEQ ID NO: 8 (VEGF-c);
SEQ ID NO: 9 (c-met); and
SEQ ID NO: 10 (claudin-4).

4. The method of claim 1, wherein said head or neck cancer is squamous cell carcinoma of the head and neck.

5. The method of claim 4, wherein said cancer is oropharyngeal or laryngeal squamous cell carcinoma.

6. The method of claim 1, wherein said biological sample is from a tumor, a cancerous tissue, a pre-cancerous tissue, a biopsy, blood, serum, saliva, or a tissue.

7. The method of claim 1, wherein said chemoradiotherapy comprises administering one or more agents selected from the group consisting of: cisplatin, cetuximab, docetaxel, and erlotinib.

8. The method of claim 7, wherein said chemoradiotherapy comprises administering cisplatin and 5-fluorouracil.

9. The method of claim 3, wherein the determination of the protein levels is carried out using immunohistochemistry, and immunoassay, a protein assay, mass spectrometry, immunofluorescence, or a combination thereof.

10. The method of claim 1, wherein the response to chemoradiotherapy treatment is whether the patient will have an incomplete response to the chemotherapy.

11. Use of a combination of YAP-1 and c-met as a biomarker for predicting the response to chemoradiotherapy treatment in a patient suffering from head or neck cancer.


SEQUENCE LISTING

<110>  William Beaumont Hospital
        Akervall, Jan

<120>  METHODS OF USING BIOMARKERS

<130>  10546-237

<140>  Not yet assigned
<141>  2009-10-22

<150>  US 61/109,331
<151>  2008-10-29

<160>  13

<170>  PatentIn version 3.5

<210>  1
<211>  5128
<212>  DNA
<213>  Homo sapiens

<400>  1
   ccgagtgcct cgcagcccct cccgaggcgc agccgccaga ccagtggagc cggggcgcag      60
   ggcgggggcg gaggcgccgg ggcgggggat gcggggccgc ggcgcagccc cccggccctg     120
   agagcgagga cagcgccgcc cggcccgcag ccgtcgccgc ttctccacct cggcccgtgg     180
   agccggggcg tccgggcgta gccctcgctc gcctgggtca gggggtgcgc gtcgggggag     240
   gcagaagcca tggatcccgg gcagcagccg ccgcctcaac cggcccccca gggccaaggg     300
   cagccgcctt cgcagccccc gcaggggcag ggcccgccgt ccggacccgg gcaaccggca     360
   cccgcggcga cccaggcggc gccgcaggca ccccccgccg ggcatcagat cgtgcacgtc     420
   cgcggggact cggagaccga cctggaggcg ctcttcaacg ccgtcatgaa ccccaagacg     480
   gccaacgtgc cccagaccgt gcccatgagg ctccggaagc tgcccgactc cttcttcaag     540
   ccgccggagc ccaaatccca ctcccgacag gccagtactg atgcaggcac tgcaggagcc     600
   ctgactccac agcatgttcg agctcattcc tctccagctt ctctgcagtt gggagctgtt     660
   tctcctggga cactgacccc cactggagta gtctctggcc cagcagctac acccacagct     720
   cagcatcttc gacagtcttc ttttgagata cctgatgatg tacctctgcc agcaggttgg     780
   gagatggcaa agacatcttc tggtcagaga tacttcttaa atcacatcga tcagacaaca     840
   acatggcagg accccaggaa ggccatgctg tcccagatga acgtcacagc ccccaccagt     900
   ccaccagtgc agcagaatat gatgaactcg gcttcagcca tgaaccagag aatcagtcag     960
   agtgctccag tgaaacagcc accacccctg gctccccaga gcccacaggg aggcgtcatg    1020
   ggtggcagca actccaacca gcagcaacag atgcgactgc agcaactgca gatggagaag    1080
   gagaggctgc ggctgaaaca gcaagaactg cttcggcagg tgaggccaca ggagttagcc    1140
   ctgcgtagcc agttaccaac actggagcag gatggtggga ctcaaaatcc agtgtcttct    1200
   cccgggatgt ctcaggaatt gagaacaatg acgaccaata gctcagatcc tttccttaac    1260
   agtggcacct atcactctcg agatgagagt acagacagtg gactaagcat gagcagctac    1320
   agtgtccctc gaaccccaga tgacttcctg aacagtgtgg atgagatgga tacaggtgat 1380
   actatcaacc aaagcaccct gccctcacag cagaaccgtt tcccagacta ccttgaagcc 1440
   attcctggga caaatgtgga ccttggaaca ctggaaggag atggaatgaa catagaagga 1500
   gaggagctga tgccaagtct gcaggaagct ttgagttctg acatccttaa tgacatggag 1560
   tctgttttgg ctgccaccaa gctagataaa gaaagctttc ttacatggtt atagagccct 1620
   caggcagact gaattctaaa tctgtgaagg atctaaggag acacatgcac cggaaatttc 1680
   cataagccag ttgcagtttt caggctaata cagaaaaaga tgaacaaacg tccagcaaga 1740
   tactttaatc ctctattttg ctcttccttg tccattgctg ctgttaatgt attgctgacc 1800
   tctttcacag ttggctctaa agaatcaaaa gaaaaaaact ttttatttct tttgctatta 1860
   aaactactgt tcattttggg ggctggggga agtgagcctg tttggatgat ggatgccatt 1920
   ccttttgccc agttaaatgt tcaccaatca ttttaactaa atactcagac ttagaagtca 1980
   gatgcttcat gtcacagcat ttagtttgtt caacagttgt ttcttcagct tcctttgtcc 2040
   agtggaaaaa catgatttac tggtctgaca agccaaaaat gttatatctg atattaaata 2100
   cttaatgctg atttgaagag atagctgaaa ccaaggctga agactgtttt actttcagta 2160
   ttttcttttc ctcctagtgc tatcattagt cacataatga ccttgatttt attttaggag 2220
   cttataaggc atgagacaat ttccatataa atatattaat tattgccaca tactctaata 2280
   tagattttgg tggataattt tgtgggtgtg cattttgttc tgttttgttg ggttttttgt 2340
   tttttttgtt tttggcaggg tcggtggggg ggttggttgg ttggttggtt ttgtcggaac 2400
   ctaggcaaat gaccatatta gtgaatctgt taatagttgt agcttgggat ggttattgta 2460
   gttgttttgg taaaatcttc atttcctggt tttttttacc accttattta aatctcgatt 2520
   atctgctctc tcttttatat acatacacac acccaaacat aacatttata atagtgtggt 2580
   agtggaatgt atcctttttt aggtttccct gctttccagt taatttttaa aatggtagcg 2640
   ctttgtatgc atttagaata catgactagt agtttatatt tcactggtag tttaaatctg 2700
   gttggggcag tctgcagatg tttgaagtag tttagtgttc tagaaagagc tattactgtg 2760
   gatagtgcct aggggagtgc tccacgccct ctgggcatac ggtagatatt atctgatgaa 2820
   ttggaaagga gcaaaccaga aatggcttta ttttctccct tggactaatt tttaagtctc 2880
   gattggaatt cagtgagtag gttcataatg tgcatgacag aaataagctt tatagtggtt 2940
   taccttcatt tagctttgga agttttcttt gccttagttt tggaagtaaa ttctagtttg 3000
   tagttctcat ttgtaatgaa cacattaacg actagattaa aatattgcct tcaagattgt 3060
   tcttacttac aagacttgct cctacttcta tgctgaaaat tgaccctgga tagaatacta 3120
   taaggttttg agttagctgg aaaagtgatc agattaataa atgtatattg gtagttgaat 3180
   ttagcaaaga aatagagata atcatgatta tacctttatt tttacaggaa gagatgatgt 3240
   aactagagta tgtgtctaca ggagtaataa tggtttccaa agagtatttt ttaaaggaac 3300
   aaaacgagca tgaattaact cttcaatata agctatgaag taatagttgg ttgtgaatta 3360
   aagtggcacc agctagcacc tctgtgtttt aagggtcttt caatgtttct agaataagcc 3420
   cttattttca agggttcata acaggcataa aatctcttct cctggcaaaa gctgctatga 3480
   aaagcctcag cttgggaaga tagatttttt tccccccaat tacaaaatct aagtattttg 3540
   gcccttcaat ttggaggagg gcaaaagttg gaagtaagaa gttttatttt aagtactttc 3600
   agtgctcaaa aaaatgcaat cactgtgttg tatataatag ttcataggtt gatcactcat 3660
   aataattgac tctaaggctt ttattaagaa aacagcagaa agattaaatc ttgaattaag 3720
   tctgggggga aatggccact gcagatggag ttttagagta gtaatgaaat tctacctaga 3780
   atgcaaaatt gggtatatga attacatagc atgttgttgg gatttttttt aatgtgcaga 3840
   agatcaaagc tacttggaag gagtgcctat aatttgccag tagccacaga ttaagattat 3900
   atcttatata tcagcagatt agctttagct tagggggagg gtgggaaagt ttgggggggg 3960
   ggttgtgaag atttaggggg accttgatag agaactttat aaacttcttt ctctttaata 4020
   aagacttgtc ttacaccgtg ctgccattaa aggcagctgt tctagagttt cagtcaccta 4080
   agtacaccca caaaacaata tgaatatgga gatcttcctt tacccctcaa ctttaatttg 4140
   cccagttata cctcagtgtt gtagcagtac tgtgatacct ggcacagtgc tttgatctta 4200
   cgatgccctc tgtactgacc tgaaggagac ctaagagtcc tttccctttt tgagtttgaa 4260
   tcatagcctt gatgtggtct cttgttttat gtccttgttc ctaatgtaaa agtgcttaac 4320
   tgcttcttgg ttgtattggg tagcattggg ataagatttt aactgggtat tcttgaattg 4380
   cttttacaat aaaccaattt tataatcttt aaatttatca actttttaca tttgtgttat 4440
   tttcagtcag ggcttcttag atctacttat ggttgatgga gcacattgat ttggagtttc 4500
   agatcttcca aagcactatt tgttgtaata acttttctaa atgtagtgcc tttaaaggaa 4560
   aaatgaacac agggaagtga ctttgctaca aataatgttg ctgtgttaag tattcatatt 4620
   aaatacatgc cttctatatg gaacatggca gaaagactga aaaataacag taattaattg 4680
   tgtaattcag aattcatacc aatcagtgtt gaaactcaaa cattgcaaaa gtgggtggca 4740
   atattcagtg cttaacactt ttctagcgtt ggtacatctg agaaatgagt gctcaggtgg 4800
   attttatcct cgcaagcatg ttgttataag aattgtgggt gtgcctatca taacaattgt 4860
   tttctgtatc ttgaaaaagt attctccaca ttttaaatgt tttatattag agaattcttt 4920
   aatgcacact tgtcaaatat atatatatag taccaatgtt acctttttat tttttgtttt 4980
   agatgtaaga gcatgctcat atgttaggta cttacataaa ttgttacatt attttttctt 5040
   atgtaatacc tttttgtttg tttatgtggt tcaaatatat tctttcctta aaaaaaaaaa 5100
   aaaaaaaaaa aaaaaaaaaa aaaaaaaa 5128

<210> 2
<211> 6030
<212> DNA
<213> Homo sapiens

<400> 2

gttggccccc gttacttttc ctctgggaaa tatggcgcac gctgggagaa cagggtacga 60
taaccgggag atagtgatga agtacatcca ttataagctg tcgcagaggg gctacgagtg 120
ggatgcggga gatgtgggcg ccgcgccccc gggggccgcc cccgcgccgg gcatcttctc 180
ctcgcagccc gggcacacgc cccatacagc cgcatcccgg gacccggtcg ccaggacctc 240
gccgctgcag accccggctg cccccggcgc cgccgcgggg cctgcgctca gcccggtgcc 300
acctgtggtc cacctgaccc tccgccaggc cggcgacgac ttctcccgcc gctaccgccg 360
cgacttcgcc gagatgtcca ggcagctgca cctgacgccc ttcaccgcgc ggggacgctt 420
tgccacggtg gtggaggagc tcttcaggga cggggtgaac tgggggagga ttgtggcctt 480
ctttgagttc ggtggggtca tgtgtgtgga gagcgtcaac cgggagatgt cgcccctggt 540
ggacaacatc gccctgtgga tgactgagta cctgaaccgg cacctgcaca cctggatcca 600
ggataacgga ggctgggatg cctttgtgga actgtacggc cccagcatgc ggcctctgtt 660
tgatttctcc tggctgtctc tgaagactct gctcagtttg gccctggtgg gagcttgcat 720
caccctgggt gcctatctgg gccacaagtg aagtcaacat gcctgcccca aacaaatatg 780
caaaaggttc actaaagcag tagaaataat atgcattgtc agtgatgttc catgaaacaa 840
agctgcaggc tgtttaagaa aaaataacac acatataaac atcacacaca cagacagaca 900
cacacacaca caacaattaa cagtcttcag gcaaaacgtc gaatcagcta tttactgcca 960
aagggaaata tcatttattt tttacattat taagaaaaaa agatttattt atttaagaca 1020
gtcccatcaa aactcctgtc tttggaaatc cgaccactaa ttgccaagca ccgcttcgtg 1080
tggctccacc tggatgttct gtgcctgtaa acatagattc gctttccatg ttgttggccg 1140
gatcaccatc tgaagagcag acggatggaa aaaggacctg atcattgggg aagctggctt 1200
tctggctgct ggaggctggg gagaaggtgt tcattcactt gcatttcttt gccctggggg 1260
ctgtgatatt aacagaggga gggttcctgt ggggggaagt ccatgcctcc ctggcctgaa 1320
gaagagactc tttgcatatg actcacatga tgcatacctg gtgggaggaa aagagttggg 1380
aacttcagat ggacctagta cccactgaga tttccacgcc gaaggacagc gatgggaaaa 1440
atgcccttaa atcataggaa agtatttttt taagctacca attgtgccga gaaaagcatt 1500
ttagcaattt atacaatatc atccagtacc ttaagccctg attgtgtata ttcatatatt 1560
ttggatacgc accccccaac tcccaatact ggctctgtct gagtaagaaa cagaatcctc 1620
tggaacttga ggaagtgaac atttcggtga cttccgcatc aggaaggcta gagttaccca 1680
gagcatcagg ccgccacaag tgcctgcttt taggagaccg aagtccgcag aacctgcctg 1740
tgtcccagct tggaggcctg gtcctggaac tgagccgggg ccctcactgg cctcctccag 1800
ggatgatcaa cagggcagtg tggtctccga atgtctggaa gctgatggag ctcagaattc 1860
cactgtcaag aaagagcagt agaggggtgt ggctgggcct gtcaccctgg ggccctccag 1920
gtaggcccgt tttcacgtgg agcatgggag ccacgaccct tcttaagaca tgtatcactg 1980
tagagggaag gaacagaggc cctgggccct tcctatcaga aggacatggt gaaggctggg 2040

aacgtgagga gaggcaatgg ccacggccca ttttggctgt agcacatggc acgttggctg 2100
tgtggccttg gcccacctgt gagtttaaag caaggcttta aatgactttg gagagggtca 2160
caaatcctaa aagaagcatt gaagtgaggt gtcatggatt aattgacccc tgtctatgga 2220
attacatgta aaacattatc ttgtcactgt agtttggttt tatttgaaaa cctgacaaaa 2280
aaaaagttcc aggtgtggaa tatgggggtt atctgtacat cctggggcat taaaaaaaaa 2340
atcaatggtg gggaactata aagaagtaac aaaagaagtg acatcttcag caaataaact 2400
aggaaatttt tttttcttcc agtttagaat cagccttgaa acattgatgg aataactctg 2460
tggcattatt gcattatata ccatttatct gtattaactt tggaatgtac tctgttcaat 2520
gtttaatgct gtggttgata tttcgaaagc tgctttaaaa aaatacatgc atctcagcgt 2580
ttttttgttt ttaattgtat ttagttatgg cctatacact atttgtgagc aaaggtgatc 2640
gttttctgtt tgagattttt atctcttgat tcttcaaaag cattctgaga aggtgagata 2700
agccctgagt ctcagctacc taagaaaaac ctggatgtca ctggccactg aggagctttg 2760
tttcaaccaa gtcatgtgca tttccacgtc aacagaattg tttattgtga cagttatatc 2820
tgttgtccct ttgaccttgt ttcttgaagg tttcctcgtc cctgggcaat tccgcattta 2880
attcatggta ttcaggatta catgcatgtt tggttaaacc catgagattc attcagttaa 2940
aaatccagat ggcaaatgac cagcagattc aaatctatgg tggtttgacc tttagagagt 3000
tgctttacgt ggcctgtttc aacacagacc cacccagagc cctcctgccc tccttccgcg 3060
ggggctttct catggctgtc cttcagggtc ttcctgaaat gcagtggtgc ttacgctcca 3120
ccaagaaagc aggaaacctg tggtatgaag ccagacctcc ccggcgggcc tcagggaaca 3180
gaatgatcag acctttgaat gattctaatt tttaagcaaa atattatttt atgaaaggtt 3240
tacattgtca aagtgatgaa tatggaatat ccaatcctgt gctgctatcc tgccaaaatc 3300
attttaatgg agtcagtttg cagtatgctc cacgtggtaa gatcctccaa gctgctttag 3360
aagtaacaat gaagaacgtg gacgctttta atataaagcc tgttttgtct tctgttgttg 3420
ttcaaacggg attcacagag tatttgaaaa atgtatatat attaagaggt cacgggggct 3480
aattgctggc tggctgcctt ttgctgtggg gttttgttac ctggttttaa taacagtaaa 3540
tgtgcccagc ctcttggccc cagaactgta cagtattgtg gctgcacttg ctctaagagt 3600
agttgatgtt gcattttcct tattgttaaa aacatgttag aagcaatgaa tgtatataaa 3660
agcctcaact agtcattttt ttctcctctt cttttttttc attatatcta attattttgc 3720
agttgggcaa cagagaacca tccctatttt gtattgaaga gggattcaca tctgcatctt 3780
aactgctctt tatgaatgaa aaaacagtcc tctgtatgta ctcctcttta cactggccag 3840
ggtcagagtt aaatagagta tatgcacttt ccaaattggg gacaagggct ctaaaaaaag 3900
ccccaaaagg agaagaacat ctgagaacct cctcggccct cccagtccct cgctgcacaa 3960
atactccgca agagaggcca gaatgacagc tgacagggtc tatggccatc gggtcgtctc 4020
cgaagatttg gcaggggcag aaaactctgg caggcttaag atttggaata aagtcacaga 4080

atcaaggaag cacctcaatt tagttcaaac aagacgccaa cattctctcc acagctcact 4140
tacctctctg tgttcagatg tggccttcca tttatatgtg atctttgttt tattagtaaa 4200
tgcttatcat ctaaagatgt agctctggcc cagtgggaaa aattaggaag tgattataaa 4260
tcgagaggag ttataataat caagattaaa tgtaaataat cagggcaatc ccaacacatg 4320
tctagctttc acctccagga tctattgagt gaacagaatt gcaaatagtc tctatttgta 4380
attgaactta tcctaaaaca aatagtttat aaatgtgaac ttaaactcta attaattcca 4440
actgtacttt taaggcagtg gctgttttta gactttctta tcacttatag ttagtaatgt 4500
acacctactc tatcagagaa aaacaggaaa ggctcgaaat acaagccatt ctaaggaaat 4560
tagggagtca gttgaaattc tattctgatc ttattctgtg gtgtcttttg cagcccagac 4620
aaatgtggtt acacactttt taagaaatac aattctacat tgtcaagctt atgaaggttc 4680
caatcagatc tttattgtta ttcaatttgg atctttcagg gatttttttt ttaaattatt 4740
atgggacaaa ggacatttgt tggaggggtg ggagggagga acaattttta aatataaaac 4800
attcccaagt ttggatcagg gagttggaag ttttcagaat aaccagaact aagggtatga 4860
aggacctgta ttggggtcga tgtgatgcct ctgcgaagaa ccttgtgtga caaatgagaa 4920
acattttgaa gtttgtggta cgacctttag attccagaga catcagcatg gctcaaagtg 4980
cagctccgtt tggcagtgca atggtataaa tttcaagctg gatatgtcta atgggtattt 5040
aaacaataaa tgtgcagttt taactaacag gatatttaat gacaaccttc tggttggtag 5100
ggacatctgt ttctaaatgt ttattatgta caatacagaa aaaaatttta taaaattaag 5160
caatgtgaaa ctgaattgga gagtgataat acaagtcctt tagtcttacc cagtgaatca 5220
ttctgttcca tgtctttgga caaccatgac cttggacaat catgaaatat gcatctcact 5280
ggatgcaaag aaaatcagat ggagcatgaa tggtactgta ccggttcatc tggactgccc 5340
cagaaaaata acttcaagca aacatcctat caacaacaag gttgttctgc ataccaagct 5400
gagcacagaa gatgggaaca ctggtggagg atggaaaggc tcgctcaatc aagaaaattc 5460
tgagactatt aataaataag actgtagtgt agatactgag taaatccatg cacctaaacc 5520
ttttggaaaa tctgccgtgg gccctccaga tagctcattt cattaagttt ttccctccaa 5580
ggtagaattt gcaagagtga cagtggattg catttctttt ggggaagctt tcttttggtg 5640
gttttgttta ttataccttc ttaagttttc aaccaaggtt tgcttttgtt ttgagttact 5700
ggggttattt ttgttttaaa taaaaataag tgtacaataa gtgtttttgt attgaaagct 5760
tttgttatca agattttcat acttttacct tccatggctc tttttaagat tgatactttt 5820
aagaggtggc tgatattctg caacactgta cacataaaaa atacggtaag gatactttac 5880
atggttaagg taaagtaagt ctccagttgg ccaccattag ctataatggc actttgtttg 5940
tgttgttgga aaaagtcaca ttgccattaa actttccttg tctgtctagt taatattgtg 6000
aagaaaaata aagtacagtg tgagatactg 6030

<210> 3

<211>   1948
<212>   DNA
<213>   Homo sapiens

<400>   3
gcggggagct cggatgtccg gtttcctgtg aggcttttac ctgacacccg ccgcctttcc      60

ccggcactgg ctgggagggc gccctgcaaa gttgggaacg cggagccccg gacccgctcc     120

cgccgcctcc ggctcgccca gggggggtcg ccgggaggag cccgggggag agggaccagg     180

aggggcccgc ggcctcgcag gggcgcccgc gcccccaccc ctgcccccgc cagcggaccg     240

gtcccccacc cccggtcctt ccaccatgca cttgctgggc ttcttctctg tggcgtgttc     300

tctgctcgcc gctgcgctgc tcccgggtcc tcgcgaggcg cccgccgccg ccgccgcctt     360

cgagtccgga ctcgacctct cggacgcgga gcccgacgcg ggcgaggcca cggcttatgc     420

aagcaaagat ctggaggagc agttacggtc tgtgtccagt gtagatgaac tcatgactgt     480

actctaccca gaatattgga aaatgtacaa gtgtcagcta aggaaaggag gctggcaaca     540

taacagagaa caggccaacc tcaactcaag gacagaagag actataaaat ttgctgcagc     600

acattataat acagagatct tgaaaagtat tgataatgag tggagaaaga ctcaatgcat     660

gccacgggag gtgtgtatag atgtggggaa ggagtttgga gtcgcgacaa acaccttctt     720

taaacctcca tgtgtgtccg tctacagatg tgggggttgc tgcaatagtg aggggctgca     780

gtgcatgaac accagcacga gctacctcag caagacgtta tttgaaatta cagtgcctct     840

ctctcaaggc cccaaaccag taacaatcag ttttgccaat cacacttcct gccgatgcat     900

gtctaaactg gatgtttaca gacaagttca ttccattatt agacgttccc tgccagcaac     960

actaccacag tgtcaggcag cgaacaagac ctgccccacc aattacatgt ggaataatca    1020

catctgcaga tgcctggctc aggaagattt tatgttttcc tcggatgctg gagatgactc    1080

aacagatgga ttccatgaca tctgtggacc aaacaaggag ctggatgaag agacctgtca    1140

gtgtgtctgc agagcggggc ttcggcctgc cagctgtgga ccccacaaag aactagacag    1200

aaactcatgc cagtgtgtct gtaaaaacaa actcttcccc agccaatgtg gggccaaccg    1260

agaatttgat gaaaacacat gccagtgtgt atgtaaaaga acctgcccca gaaatcaacc    1320

cctaaatcct ggaaaatgtg cctgtgaatg tacagaaagt ccacagaaat gcttgttaaa    1380

aggaaagaag ttccaccacc aaacatgcag ctgttacaga cggccatgta cgaaccgcca    1440

gaaggcttgt gagccaggat tttcatatag tgaagaagtg tgtcgttgtg tcccttcata    1500

ttggaaaaga ccacaaatga gctaagattg tactgttttc cagttcatcg attttctatt    1560

atggaaaact gtgttgccac agtagaactg tctgtgaaca gagagaccct tgtgggtcca    1620

tgctaacaaa gacaaaagtc tgtctttcct gaaccatgtg gataacttta cagaaatgga    1680

ctggagctca tctgcaaaag gcctcttgta aagactggtt ttctgccaat gaccaaacag    1740

ccaagatttt cctcttgtga tttctttaaa agaatgacta tataatttat ttccactaaa    1800

aatattgttt ctgcattcat ttttatagca acaacaattg gtaaaactca ctgtgatcaa    1860

tatttttata tcatgcaaaa tatgtttaaa ataaaatgaa aattgtatta taaaaaaaaa    1920

aaaaaaaaaa aaaaaaaaaa aaaaaaaa 1948

<210> 4
<211> 6641
<212> DNA
<213> Homo sapiens

<400> 4
gccctcgccg cccgcggcgc cccgagcgct ttgtgagcag atgcggagcc gagtggaggg 60

cgcgagccag atgcggggcg acagctgact tgctgagagg aggcggggag gcgcggagcg 120

cgcgtgtggt ccttgcgccg ctgacttctc cactggttcc tgggcaccga aagataaacc 180

tctcataatg aaggcccccg ctgtgcttgc acctggcatc ctcgtgctcc tgtttacctt 240

ggtgcagagg agcaatgggg agtgtaaaga ggcactagca aagtccgaga tgaatgtgaa 300

tatgaagtat cagcttccca acttcaccgc ggaaacaccc atccagaatg tcattctaca 360

tgagcatcac attttccttg gtgccactaa ctacatttat gttttaaatg aggaagacct 420

tcagaaggtt gctgagtaca agactgggcc tgtgctggaa cacccagatt gtttcccatg 480

tcaggactgc agcagcaaag ccaatttatc aggaggtgtt tggaaagata acatcaacat 540

ggctctagtt gtcgacacct actatgatga tcaactcatt agctgtggca gcgtcaacag 600

agggacctgc cagcgacatg tctttcccca caatcatact gctgacatac agtcggaggt 660

tcactgcata ttctccccac agatagaaga gcccagccag tgtcctgact gtgtggtgag 720

cgccctggga gccaaagtcc tttcatctgt aaaggaccgg ttcatcaact tctttgtagg 780

caataccata aattcttctt atttcccaga tcatccattg cattcgatat cagtgagaag 840

gctaaaggaa acgaaagatg gttttatgtt tttgacggac cagtcctaca ttgatgtttt 900

acctgagttc agagattctt accccattaa gtatgtccat gcctttgaaa gcaacaattt 960

tatttacttc ttgacggtcc aaagggaaac tctagatgct cagacttttc acacaagaat 1020

aatcaggttc tgttccataa actctggatt gcattcctac atggaaatgc ctctggagtg 1080

tattctcaca gaaaagagaa aaaagagatc cacaaagaag gaagtgttta atatacttca 1140

ggctgcgtat gtcagcaagc ctggggccca gcttgctaga caaataggag ccagcctgaa 1200

tgatgacatt cttttcgggg tgttcgcaca aagcaagcca gattctgccg aaccaatgga 1260

tcgatctgcc atgtgtgcat tccctatcaa atatgtcaac gacttcttca acaagatcgt 1320

caacaaaaac aatgtgagat gtctccagca tttttacgga cccaatcatg agcactgctt 1380

taataggaca cttctgagaa attcatcagg ctgtgaagcg cgccgtgatg aatatcgaac 1440

agagtttacc acagctttgc agcgcgttga cttattcatg ggtcaattca gcgaagtcct 1500

cttaacatct atatccacct tcattaaagg agacctcacc atagctaatc ttgggacatc 1560

agagggtcgc ttcatgcagg ttgtggtttc tcgatcagga ccatcaaccc ctcatgtgaa 1620

ttttctcctg gactcccatc cagtgtctcc agaagtgatt gtggagcata cattaaacca 1680

aaatggctac acactggtta tcactgggaa gaagatcacg aagatcccat tgaatggctt 1740

gggctgcaga catttccagt cctgcagtca atgcctctct gccccaccct ttgttcagtg 1800
tggctggtgc cacgacaaat gtgtgcgatc ggaggaatgc ctgagcggga catggactca 1860
acagatctgt ctgcctgcaa tctacaaggt tttcccaaat agtgcacccc ttgaaggagg 1920
gacaaggctg accatatgtg gctgggactt tggatttcgg aggaataata aatttgattt 1980
aaagaaaact agagttctcc ttggaaatga gagctgcacc ttgactttaa gtgagagcac 2040
gatgaataca ttgaaatgca cagttggtcc tgccatgaat aagcatttca atatgtccat 2100
aattatttca aatggccacg ggacaacaca atacagtaca ttctcctatg tggatcctgt 2160
aataacaagt atttcgccga aatacggtcc tatggctggt ggcactttac ttactttaac 2220
tggaaattac ctaaacagtg ggaattctag acacatttca attggtggaa aaacatgtac 2280
tttaaaaagt gtgtcaaaca gtattcttga atgttatacc ccagcccaaa ccatttcaac 2340
tgagtttgct gttaaattga aaattgactt agccaaccga gagacaagca tcttcagtta 2400
ccgtgaagat cccattgtct atgaaattca tccaaccaaa tcttttatta gtggtgggag 2460
cacaataaca ggtgttggga aaaacctgaa ttcagttagt gtcccgagaa tggtcataaa 2520
tgtgcatgaa gcaggaagga actttacagt ggcatgtcaa catcgctcta attcagagat 2580
aatctgttgt accactcctt ccctgcaaca gctgaatctg caactccccc tgaaaaccaa 2640
agcctttttc atgttagatg ggatcctttc caaatacttt gatctcattt atgtacataa 2700
tcctgtgttt aagccttttg aaaagccagt gatgatctca atgggcaatg aaaatgtact 2760
ggaaattaag ggaaatgata ttgaccctga agcagttaaa ggtgaagtgt taaaagttgg 2820
aaataagagc tgtgagaata tacacttaca ttctgaagcc gttttatgca cggtccccaa 2880
tgacctgctg aaattgaaca gcgagctaaa tatagagtgg aagcaagcaa tttcttcaac 2940
cgtccttgga aaagtaatag ttcaaccaga tcagaatttc acaggattga ttgctggtgt 3000
tgtctcaata tcaacagcac tgttattact acttgggttt ttcctgtggc tgaaaaagag 3060
aaagcaaatt aaagatctgg gcagtgaatt agttcgctac gatgcaagag tacacactcc 3120
tcatttggat aggcttgtaa gtgcccgaag tgtaagccca actacagaaa tggtttcaaa 3180
tgaatctgta gactaccgag ctacttttcc agaagatcag tttcctaatt catctcagaa 3240
cggttcatgc cgacaagtgc agtatcctct gacagacatg tcccccatcc taactagtgg 3300
ggactctgat atatccagtc cattactgca aaatactgtc cacattgacc tcagtgctct 3360
aaatccagag ctggtccagg cagtgcagca tgtagtgatt gggcccagta gcctgattgt 3420
gcatttcaat gaagtcatag gaagagggca ttttggttgt gtatatcatg ggactttgtt 3480
ggacaatgat ggcaagaaaa ttcactgtgc tgtgaaatcc ttgaacagaa tcactgacat 3540
aggagaagtt tcccaatttc tgaccgaggg aatcatcatg aaagatttta gtcatcccaa 3600
tgtcctctcg ctcctgggaa tctgcctgcg aagtgaaggg tctccgctgg tggtcctacc 3660
atacatgaaa catggagatc ttcgaaattt cattcgaaat gagactcata atccaactgt 3720
aaaagatctt attggctttg gtcttcaagt agccaaaggc atgaaatatc ttgcaagcaa 3780

aaagtttgtc cacagagact tggctgcaag aaactgtatg ctggatgaaa aattcacagt 3840
caaggttgct gattttggtc ttgccagaga catgtatgat aaagaatact atagtgtaca 3900
caacaaaaca ggtgcaaagc tgccagtgaa gtggatggct ttggaaagtc tgcaaactca 3960
aaagtttacc accaagtcag atgtgtggtc ctttggcgtg ctcctctggg agctgatgac 4020
aagaggagcc ccaccttatc ctgacgtaaa cacctttgat ataactgttt acttgttgca 4080
agggagaaga ctcctacaac ccgaatactg cccagacccc ttatatgaag taatgctaaa 4140
atgctggcac cctaaagccg aaatgcgccc atccttttct gaactggtgt cccggatatc 4200
agcgatcttc tctactttca ttggggagca ctatgtccat gtgaacgcta cttatgtgaa 4260
cgtaaaatgt gtcgctccgt atccttctct gttgtcatca gaagataacg ctgatgatga 4320
ggtggacaca cgaccagcct ccttctggga gacatcatag tgctagtact atgtcaaagc 4380
aacagtccac actttgtcca atggtttttt cactgcctga cctttaaaag gccatcgata 4440
ttctttgctc ttgccaaaat tgcactatta taggacttgt attgttattt aaattactgg 4500
attctaagga atttcttatc tgacagagca tcagaaccag aggcttggtc ccacaggcca 4560
cggaccaatg gcctgcagcc gtgacaacac tcctgtcata ttggagtcca aaacttgaat 4620
tctgggttga attttttaaa aatcaggtac cacttgattt catatgggaa attgaagcag 4680
gaaatattga gggcttcttg atcacagaaa actcagaaga gatagtaatg ctcaggacag 4740
gagcggcagc cccagaacag gccactcatt tagaattcta gtgtttcaaa acacttttgt 4800
gtgttgtatg gtcaataaca tttttcatta ctgatggtgt cattcaccca ttaggtaaac 4860
attccctttt aaatgtttgt ttgttttttg agacaggatc tcactctgtt gccagggctg 4920
tagtgcagtg gtgtgatcat agctcactgc aacctccacc tcccaggctc aagcctcccg 4980
aatagctggg actacaggcg cacaccacca tccccggcta atttttgtat tttttgtaga 5040
gacggggttt tgccatgttg ccaaggctgg tttcaaactc ctggactcaa gaaatccacc 5100
cacctcagcc tcccaaagtg ctaggattac aggcatgagc cactgcgccc agcccttata 5160
aatttttgta tagacattcc tttggttgga agaatattta taggcaatac agtcaaagtt 5220
tcaaaatagc atcacacaaa acatgtttat aaatgaacag gatgtaatgt acatagatga 5280
cattaagaaa atttgtatga aataatttag tcatcatgaa atatttagtt gtcatataaa 5340
aacccactgt ttgagaatga tgctactctg atctaatgaa tgtgaacatg tagatgtttt 5400
gtgtgtattt ttttaaatga aaactcaaaa taagacaagt aatttgttga taaatatttt 5460
taaagataac tcagcatgtt tgtaaagcag gatacatttt actaaaaggt tcattggttc 5520
caatcacagc tcataggtag agcaaagaaa gggtggatgg attgaaaaga ttagcctctg 5580
tctcggtggc aggttcccac ctcgcaagca attggaaaca aaacttttgg ggagttttat 5640
tttgcattag ggtgtgtttt atgttaagca aaacatactt tagaaacaaa tgaaaaaggc 5700
aattgaaaat cccagctatt tcacctagat ggaatagcca ccctgagcag aactttgtga 5760
tgcttcattc tgtggaattt tgtgcttgct actgtatagt gcatgtggtg taggttactc 5820

taactggttt tgtcgacgta aacatttaaa gtgttatatt ttttataaaa atgtttattt 5880

ttaatgatat gagaaaaatt ttgttaggcc acaaaaacac tgcactgtga acattttaga 5940

aaaggtatgt cagactggga ttaatgacag catgattttc aatgactgta aattgcgata 6000

aggaaatgta ctgattgcca atacacccca ccctcattac atcatcagga cttgaagcca 6060

agggttaacc cagcaagcta caaagagggt gtgtcacact gaaactcaat agttgagttt 6120

ggctgttgtt gcaggaaaat gattataact aaaagctctc tgatagtgca gagacttacc 6180

agaagacaca aggaattgta ctgaagagct attacaatcc aaatattgcc gtttcataaa 6240

tgtaataagt aatactaatt cacagagtat tgtaaatggt ggatgacaaa agaaaatctg 6300

ctctgtggaa agaaagaact gtctctacca gggtcaagag catgaacgca tcaatagaaa 6360

gaactcgggg aaacatccca tcaacaggac tacacacttg tatatacatt cttgagaaca 6420

ctgcaatgtg aaaatcacgt ttgctattta taaacttgtc cttagattaa tgtgtctgga 6480

cagattgtgg gagtaagtga ttcttctaag aattagatac ttgtcactgc ctatacctgc 6540

agctgaactg aatggtactt cgtatgttaa tagttgttct gataaatcat gcaattaaag 6600

taaagtgatg caacatcttg taaaaaaaaa aaaaaaaaaa a 6641

<210> 5
<211> 1846
<212> DNA
<213> Homo sapiens

<400> 5
aaaagtgcct ttgttggcct gggctcagga atccagagaa actggtcagg aggaggcccc 60

agtgacaaaa acccctccct ctgcccccgc ccctctgcca gagccatata actgctcaac 120

ctgtccccga gagagagtgc cctggcagct gtcggctgga aggaactggt ctgctcacac 180

ttgctggctt gcgcatcagg actggcttta tctcctgact cacggtgcaa aggtgcactc 240

tgcgaacgtt aagtccgtcc ccagcgcttg gaatcctacg gcccccacag ccggatcccc 300

tcagccttcc aggtcctcaa ctcccgtgga cgctgaacaa tggcctccat ggggctacag 360

gtaatgggca tcgcgctggc cgtcctgggc tggctggccg tcatgctgtg ctgcgcgctg 420

cccatgtggc gcgtgacggc cttcatcggc agcaacattg tcacctcgca gaccatctgg 480

gagggcctat ggatgaactg cgtggtgcag agcaccggcc agatgcagtg caaggtgtac 540

gactcgctgc tggcactgcc gcaggacctg caggcggccc gcgccctcgt catcatcagc 600

atcatcgtgg ctgctctggg cgtgctgctg tccgtggtgg ggggcaagtg taccaactgc 660

ctggaggatg aaagcgccaa ggccaagacc atgatcgtgg cgggcgtggt gttcctgttg 720

gccggcctta tggtgatagt gccggtgtcc tggacggccc acaacatcat ccaagacttc 780

tacaatccgc tggtggcctc cgggcagaag cgggagatgg gtgcctcgct ctacgtcggc 840

tgggccgcct ccggcctgct gctccttggc ggggggctgc tttgctgcaa ctgtccaccc 900

cgcacagaca agccttactc cgccaagtat tctgctgccc gctctgctgc tgccagcaac 960

tacgtgtaag gtgccacggc tccactctgt tcctctctgc tttgttcttc cctggactga 1020

gctcagcgca ggctgtgacc ccaggagggc cctgccacgg gccactggct gctggggact 1080

ggggactggg cagagactga gccaggcagg aaggcagcag ccttcagcct ctctggccca 1140

ctcggacaac ttcccaaggc cgcctcctgc tagcaagaac agagtccacc ctcctctgga 1200

tattggggag ggacggaagt gacagggtgt ggtggtggag tggggagctg gcttctgctg 1260

gccaggatag cttaaccctg actttgggat ctgcctgcat cggcgttggc cactgtcccc 1320

atttacattt tccccactct gtctgcctgc atctcctctg ttccgggtag gccttgatat 1380

cacctctggg actgtgcctt gctcaccgaa acccgcgccc aggagtatgg ctgaggcctt 1440

gcccacccac ctgcctggga agtgcagagt ggatggacgg gtttagaggg gaggggcgaa 1500

ggtgctgtaa acaggtttgg gcagtggtgg gggagggggc cagagaggcg gctcaggttg 1560

cccagctctg tggcctcagg actctctgcc tcacccgctt cagcccaggg cccctggaga 1620

ctgatcccct ctgagtcctc tgccccttcc aaggacacta atgagcctgg gagggtggca 1680

gggaggaggg gacagcttca cccttggaag tcctggggtt tttcctcttc cttctttgtg 1740

gtttctgttt tgtaatttaa gaagagctat tcatcactgt aattattatt attttctaca 1800

ataaatggga cctgtgcaca ggaaaaaaaa aaaaaaaaaa aaaaaa 1846

<210> 6
<211> 454
<212> PRT
<213> Homo sapiens

<400> 6

Met Asp Pro Gly Gln Gln Pro Pro Pro Gln Pro Ala Pro Gln Gly Gln
1               5                   10                  15

Gly Gln Pro Pro Ser Gln Pro Pro Gln Gly Gln Gly Pro Pro Ser Gly
            20                  25                  30

Pro Gly Gln Pro Ala Pro Ala Ala Thr Gln Ala Ala Pro Gln Ala Pro
        35                  40                  45

Pro Ala Gly His Gln Ile Val His Val Arg Gly Asp Ser Glu Thr Asp
    50                  55                  60

Leu Glu Ala Leu Phe Asn Ala Val Met Asn Pro Lys Thr Ala Asn Val
65                  70                  75                  80

Pro Gln Thr Val Pro Met Arg Leu Arg Lys Leu Pro Asp Ser Phe Phe
                85                  90                  95

Lys Pro Pro Glu Pro Lys Ser His Ser Arg Gln Ala Ser Thr Asp Ala
            100                 105                 110

Gly Thr Ala Gly Ala Leu Thr Pro Gln His Val Arg Ala His Ser Ser
        115                 120                 125

Pro Ala Ser Leu Gln Leu Gly Ala Val Ser Pro Gly Thr Leu Thr Pro
    130                 135                 140

Thr Gly Val Val Ser Gly Pro Ala Ala Thr Pro Thr Ala Gln His Leu
145                 150                 155                 160

Arg Gln Ser Ser Phe Glu Ile Pro Asp Asp Val Pro Leu Pro Ala Gly
                165                 170                 175

Trp Glu Met Ala Lys Thr Ser Ser Gly Gln Arg Tyr Phe Leu Asn His
            180                 185                 190

Ile Asp Gln Thr Thr Thr Trp Gln Asp Pro Arg Lys Ala Met Leu Ser
        195                 200                 205

Gln Met Asn Val Thr Ala Pro Thr Ser Pro Pro Val Gln Gln Asn Met
    210                 215                 220

Met Asn Ser Ala Ser Ala Met Asn Gln Arg Ile Ser Gln Ser Ala Pro
225                 230                 235                 240

Val Lys Gln Pro Pro Pro Leu Ala Pro Gln Ser Pro Gln Gly Gly Val
                245                 250                 255

Met Gly Gly Ser Asn Ser Asn Gln Gln Gln Gln Met Arg Leu Gln Gln
            260                 265                 270

Leu Gln Met Glu Lys Glu Arg Leu Arg Leu Lys Gln Gln Glu Leu Leu
        275                 280                 285

Arg Gln Val Arg Pro Gln Glu Leu Ala Leu Arg Ser Gln Leu Pro Thr
    290                 295                 300

Leu Glu Gln Asp Gly Gly Thr Gln Asn Pro Val Ser Ser Pro Gly Met
305                 310                 315                 320

Ser Gln Glu Leu Arg Thr Met Thr Thr Asn Ser Ser Asp Pro Phe Leu
                325                 330                 335

Asn Ser Gly Thr Tyr His Ser Arg Asp Glu Ser Thr Asp Ser Gly Leu
            340                 345                 350

Ser Met Ser Ser Tyr Ser Val Pro Arg Thr Pro Asp Asp Phe Leu Asn
        355                 360                 365

Ser Val Asp Glu Met Asp Thr Gly Asp Thr Ile Asn Gln Ser Thr Leu
    370                 375                 380

Pro Ser Gln Gln Asn Arg Phe Pro Asp Tyr Leu Glu Ala Ile Pro Gly
385                 390                 395                 400

Thr Asn Val Asp Leu Gly Thr Leu Glu Gly Asp Gly Met Asn Ile Glu
                405                 410                 415

Gly Glu Glu Leu Met Pro Ser Leu Gln Glu Ala Leu Ser Ser Asp Ile
            420                 425                 430

Leu Asn Asp Met Glu Ser Val Leu Ala Ala Thr Lys Leu Asp Lys Glu
        435                 440                 445

Ser Phe Leu Thr Trp Leu
    450

<210> 7
<211> 239
<212> PRT
<213> Homo sapiens

<400> 7

Met Ala His Ala Gly Arg Thr Gly Tyr Asp Asn Arg Glu Ile Val Met
1               5                   10                  15

Lys Tyr Ile His Tyr Lys Leu Ser Gln Arg Gly Tyr Glu Trp Asp Ala
            20                  25                  30

Gly Asp Val Gly Ala Ala Pro Pro Gly Ala Ala Pro Ala Pro Gly Ile
        35                  40                  45

Phe Ser Ser Gln Pro Gly His Thr Pro His Thr Ala Ala Ser Arg Asp
    50                  55                  60

Pro Val Ala Arg Thr Ser Pro Leu Gln Thr Pro Ala Ala Pro Gly Ala
65                  70                  75                  80

Ala Ala Gly Pro Ala Leu Ser Pro Val Pro Pro Val Val His Leu Thr
                85                  90                  95

Leu Arg Gln Ala Gly Asp Asp Phe Ser Arg Arg Tyr Arg Arg Asp Phe
            100                 105                 110

Ala Glu Met Ser Arg Gln Leu His Leu Thr Pro Phe Thr Ala Arg Gly
        115                 120                 125

Arg Phe Ala Thr Val Val Glu Glu Leu Phe Arg Asp Gly Val Asn Trp
    130                 135                 140

Gly Arg Ile Val Ala Phe Phe Glu Phe Gly Gly Val Met Cys Val Glu
145                 150                 155                 160

Ser Val Asn Arg Glu Met Ser Pro Leu Val Asp Asn Ile Ala Leu Trp
                165                 170                 175

Met Thr Glu Tyr Leu Asn Arg His Leu His Thr Trp Ile Gln Asp Asn
            180                 185                 190

Gly Gly Trp Asp Ala Phe Val Glu Leu Tyr Gly Pro Ser Met Arg Pro
        195                 200                 205

Leu Phe Asp Phe Ser Trp Leu Ser Leu Lys Thr Leu Leu Ser Leu Ala
    210                 215                 220

Leu Val Gly Ala Cys Ile Thr Leu Gly Ala Tyr Leu Gly His Lys
225                 230                 235

<210>  8
<211>  419
<212>  PRT
<213>  Homo sapiens

<400>  8

Met His Leu Leu Gly Phe Phe Ser Val Ala Cys Ser Leu Leu Ala Ala
1               5                   10                  15

Ala Leu Leu Pro Gly Pro Arg Glu Ala Pro Ala Ala Ala Ala Ala Phe
            20                  25                  30

Glu Ser Gly Leu Asp Leu Ser Asp Ala Glu Pro Asp Ala Gly Glu Ala
        35                  40                  45

Thr Ala Tyr Ala Ser Lys Asp Leu Glu Glu Gln Leu Arg Ser Val Ser
    50                  55                  60

Ser Val Asp Glu Leu Met Thr Val Leu Tyr Pro Glu Tyr Trp Lys Met
65                  70                  75                  80

Tyr Lys Cys Gln Leu Arg Lys Gly Gly Trp Gln His Asn Arg Glu Gln
                85                  90                  95

Ala Asn Leu Asn Ser Arg Thr Glu Glu Thr Ile Lys Phe Ala Ala Ala
            100                 105                 110

His Tyr Asn Thr Glu Ile Leu Lys Ser Ile Asp Asn Glu Trp Arg Lys
        115                 120                 125

Thr Gln Cys Met Pro Arg Glu Val Cys Ile Asp Val Gly Lys Glu Phe
    130                 135                 140

Gly Val Ala Thr Asn Thr Phe Phe Lys Pro Pro Cys Val Ser Val Tyr
145                 150                 155                 160

Arg Cys Gly Gly Cys Cys Asn Ser Glu Gly Leu Gln Cys Met Asn Thr
                165                 170                 175

Ser Thr Ser Tyr Leu Ser Lys Thr Leu Phe Glu Ile Thr Val Pro Leu

180                 185                 190

Ser Gln Gly Pro Lys Pro Val Thr Ile Ser Phe Ala Asn His Thr Ser
        195                 200                 205

Cys Arg Cys Met Ser Lys Leu Asp Val Tyr Arg Gln Val His Ser Ile
    210                 215                 220

Ile Arg Arg Ser Leu Pro Ala Thr Leu Pro Gln Cys Gln Ala Ala Asn
225                 230                 235                 240

Lys Thr Cys Pro Thr Asn Tyr Met Trp Asn Asn His Ile Cys Arg Cys
                245                 250                 255

Leu Ala Gln Glu Asp Phe Met Phe Ser Ser Asp Ala Gly Asp Asp Ser
            260                 265                 270

Thr Asp Gly Phe His Asp Ile Cys Gly Pro Asn Lys Glu Leu Asp Glu
        275                 280                 285

Glu Thr Cys Gln Cys Val Cys Arg Ala Gly Leu Arg Pro Ala Ser Cys
    290                 295                 300

Gly Pro His Lys Glu Leu Asp Arg Asn Ser Cys Gln Cys Val Cys Lys
305                 310                 315                 320

Asn Lys Leu Phe Pro Ser Gln Cys Gly Ala Asn Arg Glu Phe Asp Glu
                325                 330                 335

Asn Thr Cys Gln Cys Val Cys Lys Arg Thr Cys Pro Arg Asn Gln Pro
            340                 345                 350

Leu Asn Pro Gly Lys Cys Ala Cys Glu Cys Thr Glu Ser Pro Gln Lys
        355                 360                 365

Cys Leu Leu Lys Gly Lys Lys Phe His His Gln Thr Cys Ser Cys Tyr
    370                 375                 380

Arg Arg Pro Cys Thr Asn Arg Gln Lys Ala Cys Glu Pro Gly Phe Ser
385                 390                 395                 400

Tyr Ser Glu Glu Val Cys Arg Cys Val Pro Ser Tyr Trp Lys Arg Pro
                405                 410                 415

Gln Met Ser

<210>  9
<211>  1390
<212>  PRT
<213>  Homo sapiens

<400> 9

Met Lys Ala Pro Ala Val Leu Ala Pro Gly Ile Leu Val Leu Leu Phe
1               5                   10                  15

Thr Leu Val Gln Arg Ser Asn Gly Glu Cys Lys Glu Ala Leu Ala Lys
            20                  25                  30

Ser Glu Met Asn Val Asn Met Lys Tyr Gln Leu Pro Asn Phe Thr Ala
        35                  40                  45

Glu Thr Pro Ile Gln Asn Val Ile Leu His Glu His His Ile Phe Leu
    50                  55                  60

Gly Ala Thr Asn Tyr Ile Tyr Val Leu Asn Glu Glu Asp Leu Gln Lys
65                  70                  75                  80

Val Ala Glu Tyr Lys Thr Gly Pro Val Leu Glu His Pro Asp Cys Phe
                85                  90                  95

Pro Cys Gln Asp Cys Ser Ser Lys Ala Asn Leu Ser Gly Gly Val Trp
            100                 105                 110

Lys Asp Asn Ile Asn Met Ala Leu Val Val Asp Thr Tyr Tyr Asp Asp
        115                 120                 125

Gln Leu Ile Ser Cys Gly Ser Val Asn Arg Gly Thr Cys Gln Arg His
    130                 135                 140

Val Phe Pro His Asn His Thr Ala Asp Ile Gln Ser Glu Val His Cys
145                 150                 155                 160

Ile Phe Ser Pro Gln Ile Glu Glu Pro Ser Gln Cys Pro Asp Cys Val
                165                 170                 175

Val Ser Ala Leu Gly Ala Lys Val Leu Ser Ser Val Lys Asp Arg Phe
            180                 185                 190

Ile Asn Phe Phe Val Gly Asn Thr Ile Asn Ser Ser Tyr Phe Pro Asp
        195                 200                 205

His Pro Leu His Ser Ile Ser Val Arg Arg Leu Lys Glu Thr Lys Asp
    210                 215                 220

Gly Phe Met Phe Leu Thr Asp Gln Ser Tyr Ile Asp Val Leu Pro Glu
225                 230                 235                 240

Phe Arg Asp Ser Tyr Pro Ile Lys Tyr Val His Ala Phe Glu Ser Asn
                245                 250                 255

Asn Phe Ile Tyr Phe Leu Thr Val Gln Arg Glu Thr Leu Asp Ala Gln
            260                 265                 270

Thr Phe His Thr Arg Ile Ile Arg Phe Cys Ser Ile Asn Ser Gly Leu
        275                 280                 285

His Ser Tyr Met Glu Met Pro Leu Glu Cys Ile Leu Thr Glu Lys Arg
    290                 295                 300

Lys Lys Arg Ser Thr Lys Lys Glu Val Phe Asn Ile Leu Gln Ala Ala
305                 310                 315                 320

Tyr Val Ser Lys Pro Gly Ala Gln Leu Ala Arg Gln Ile Gly Ala Ser
                325                 330                 335

Leu Asn Asp Asp Ile Leu Phe Gly Val Phe Ala Gln Ser Lys Pro Asp
            340                 345                 350

Ser Ala Glu Pro Met Asp Arg Ser Ala Met Cys Ala Phe Pro Ile Lys
        355                 360                 365

Tyr Val Asn Asp Phe Phe Asn Lys Ile Val Asn Lys Asn Asn Val Arg
    370                 375                 380

Cys Leu Gln His Phe Tyr Gly Pro Asn His Glu His Cys Phe Asn Arg
385                 390                 395                 400

Thr Leu Leu Arg Asn Ser Ser Gly Cys Glu Ala Arg Arg Asp Glu Tyr
                405                 410                 415

Arg Thr Glu Phe Thr Thr Ala Leu Gln Arg Val Asp Leu Phe Met Gly
            420                 425                 430

Gln Phe Ser Glu Val Leu Leu Thr Ser Ile Ser Thr Phe Ile Lys Gly
        435                 440                 445

Asp Leu Thr Ile Ala Asn Leu Gly Thr Ser Glu Gly Arg Phe Met Gln
    450                 455                 460

Val Val Val Ser Arg Ser Gly Pro Ser Thr Pro His Val Asn Phe Leu
465                 470                 475                 480

Leu Asp Ser His Pro Val Ser Pro Glu Val Ile Val Glu His Thr Leu
                485                 490                 495

Asn Gln Asn Gly Tyr Thr Leu Val Ile Thr Gly Lys Lys Ile Thr Lys
            500                 505                 510

Ile Pro Leu Asn Gly Leu Gly Cys Arg His Phe Gln Ser Cys Ser Gln
        515                 520                 525

Cys Leu Ser Ala Pro Pro Phe Val Gln Cys Gly Trp Cys His Asp Lys
    530                 535                 540

Cys Val Arg Ser Glu Glu Cys Leu Ser Gly Thr Trp Thr Gln Gln Ile
545                 550                 555                 560

Cys Leu Pro Ala Ile Tyr Lys Val Phe Pro Asn Ser Ala Pro Leu Glu
                565                 570                 575

Gly Gly Thr Arg Leu Thr Ile Cys Gly Trp Asp Phe Gly Phe Arg Arg
            580                 585                 590

Asn Asn Lys Phe Asp Leu Lys Lys Thr Arg Val Leu Leu Gly Asn Glu
        595                 600                 605

Ser Cys Thr Leu Thr Leu Ser Glu Ser Thr Met Asn Thr Leu Lys Cys
    610                 615                 620

Thr Val Gly Pro Ala Met Asn Lys His Phe Asn Met Ser Ile Ile Ile
625                 630                 635                 640

Ser Asn Gly His Gly Thr Thr Gln Tyr Ser Thr Phe Ser Tyr Val Asp
                645                 650                 655

Pro Val Ile Thr Ser Ile Ser Pro Lys Tyr Gly Pro Met Ala Gly Gly
            660                 665                 670

Thr Leu Leu Thr Leu Thr Gly Asn Tyr Leu Asn Ser Gly Asn Ser Arg
        675                 680                 685

His Ile Ser Ile Gly Gly Lys Thr Cys Thr Leu Lys Ser Val Ser Asn
    690                 695                 700

Ser Ile Leu Glu Cys Tyr Thr Pro Ala Gln Thr Ile Ser Thr Glu Phe
705                 710                 715                 720

Ala Val Lys Leu Lys Ile Asp Leu Ala Asn Arg Glu Thr Ser Ile Phe
                725                 730                 735

Ser Tyr Arg Glu Asp Pro Ile Val Tyr Glu Ile His Pro Thr Lys Ser
            740                 745                 750

Phe Ile Ser Gly Gly Ser Thr Ile Thr Gly Val Gly Lys Asn Leu Asn
        755                 760                 765

Ser Val Ser Val Pro Arg Met Val Ile Asn Val His Glu Ala Gly Arg
    770                 775                 780

Asn Phe Thr Val Ala Cys Gln His Arg Ser Asn Ser Glu Ile Ile Cys
785                 790                 795                 800

Cys Thr Thr Pro Ser Leu Gln Gln Leu Asn Leu Gln Leu Pro Leu Lys
                805                 810                 815

Thr Lys Ala Phe Phe Met Leu Asp Gly Ile Leu Ser Lys Tyr Phe Asp
            820                 825                 830

Leu Ile Tyr Val His Asn Pro Val Phe Lys Pro Phe Glu Lys Pro Val
        835                 840                 845

Met Ile Ser Met Gly Asn Glu Asn Val Leu Glu Ile Lys Gly Asn Asp
    850                 855                 860

Ile Asp Pro Glu Ala Val Lys Gly Glu Val Leu Lys Val Gly Asn Lys
865                 870                 875                 880

Ser Cys Glu Asn Ile His Leu His Ser Glu Ala Val Leu Cys Thr Val
                885                 890                 895

Pro Asn Asp Leu Leu Lys Leu Asn Ser Glu Leu Asn Ile Glu Trp Lys
            900                 905                 910

Gln Ala Ile Ser Ser Thr Val Leu Gly Lys Val Ile Val Gln Pro Asp
        915                 920                 925

Gln Asn Phe Thr Gly Leu Ile Ala Gly Val Val Ser Ile Ser Thr Ala
    930                 935                 940

Leu Leu Leu Leu Leu Gly Phe Phe Leu Trp Leu Lys Lys Arg Lys Gln
945                 950                 955                 960

Ile Lys Asp Leu Gly Ser Glu Leu Val Arg Tyr Asp Ala Arg Val His
                965                 970                 975

Thr Pro His Leu Asp Arg Leu Val Ser Ala Arg Ser Val Ser Pro Thr
            980                 985                 990

Thr Glu Met Val Ser Asn Glu Ser  Val Asp Tyr Arg Ala  Thr Phe Pro
        995                 1000                1005

Glu Asp  Gln Phe Pro Asn Ser  Ser Gln Asn Gly Ser  Cys Arg Gln
    1010                1015                1020

Val Gln  Tyr Pro Leu Thr Asp  Met Ser Pro Ile Leu  Thr Ser Gly
    1025                1030                1035

Asp Ser  Asp Ile Ser Ser Pro  Leu Leu Gln Asn Thr  Val His Ile
    1040                1045                1050

Asp Leu  Ser Ala Leu Asn Pro  Glu Leu Val Gln Ala  Val Gln His
    1055                1060                1065

Val Val  Ile Gly Pro Ser Ser  Leu Ile Val His Phe  Asn Glu Val
    1070                1075                1080

Ile Gly Arg Gly His Phe Gly Cys Val Tyr His Gly Thr Leu Leu
    1085                1090                1095

Asp Asn Asp Gly Lys Lys Ile His Cys Ala Val Lys Ser Leu Asn
    1100                1105                1110

Arg Ile Thr Asp Ile Gly Glu Val Ser Gln Phe Leu Thr Glu Gly
    1115                1120                1125

Ile Ile Met Lys Asp Phe Ser His Pro Asn Val Leu Ser Leu Leu
    1130                1135                1140

Gly Ile Cys Leu Arg Ser Glu Gly Ser Pro Leu Val Val Leu Pro
    1145                1150                1155

Tyr Met Lys His Gly Asp Leu Arg Asn Phe Ile Arg Asn Glu Thr
    1160                1165                1170

His Asn Pro Thr Val Lys Asp Leu Ile Gly Phe Gly Leu Gln Val
    1175                1180                1185

Ala Lys Gly Met Lys Tyr Leu Ala Ser Lys Lys Phe Val His Arg
    1190                1195                1200

Asp Leu Ala Ala Arg Asn Cys Met Leu Asp Glu Lys Phe Thr Val
    1205                1210                1215

Lys Val Ala Asp Phe Gly Leu Ala Arg Asp Met Tyr Asp Lys Glu
    1220                1225                1230

Tyr Tyr Ser Val His Asn Lys Thr Gly Ala Lys Leu Pro Val Lys
    1235                1240                1245

Trp Met Ala Leu Glu Ser Leu Gln Thr Gln Lys Phe Thr Thr Lys
    1250                1255                1260

Ser Asp Val Trp Ser Phe Gly Val Leu Leu Trp Glu Leu Met Thr
    1265                1270                1275

Arg Gly Ala Pro Pro Tyr Pro Asp Val Asn Thr Phe Asp Ile Thr
    1280                1285                1290

Val Tyr Leu Leu Gln Gly Arg Arg Leu Leu Gln Pro Glu Tyr Cys
    1295                1300                1305

Pro Asp Pro Leu Tyr Glu Val Met Leu Lys Cys Trp His Pro Lys
    1310                1315                1320

Ala Glu Met Arg Pro Ser Phe Ser Glu Leu Val Ser Arg Ile Ser
    1325                1330                1335

Ala Ile Phe Ser Thr Phe Ile Gly Glu His Tyr Val His Val Asn
    1340                1345                1350

Ala Thr Tyr Val Asn Val Lys Cys Val Ala Pro Tyr Pro Ser Leu
    1355                1360                1365

Leu Ser Ser Glu Asp Asn Ala Asp Asp Glu Val Asp Thr Arg Pro
    1370                1375                1380

Ala Ser Phe Trp Glu Thr Ser
    1385                1390

<210> 10
<211> 209
<212> PRT
<213> Homo sapiens

<400> 10

Met Ala Ser Met Gly Leu Gln Val Met Gly Ile Ala Leu Ala Val Leu
1               5                   10                  15

Gly Trp Leu Ala Val Met Leu Cys Cys Ala Leu Pro Met Trp Arg Val
            20                  25                  30

Thr Ala Phe Ile Gly Ser Asn Ile Val Thr Ser Gln Thr Ile Trp Glu
        35                  40                  45

Gly Leu Trp Met Asn Cys Val Val Gln Ser Thr Gly Gln Met Gln Cys
    50                  55                  60

Lys Val Tyr Asp Ser Leu Leu Ala Leu Pro Gln Asp Leu Gln Ala Ala
65                  70                  75                  80

Arg Ala Leu Val Ile Ile Ser Ile Ile Val Ala Ala Leu Gly Val Leu
                85                  90                  95

Leu Ser Val Val Gly Gly Lys Cys Thr Asn Cys Leu Glu Asp Glu Ser
            100                 105                 110

Ala Lys Ala Lys Thr Met Ile Val Ala Gly Val Val Phe Leu Leu Ala
        115                 120                 125

Gly Leu Met Val Ile Val Pro Val Ser Trp Thr Ala His Asn Ile Ile
    130                 135                 140

Gln Asp Phe Tyr Asn Pro Leu Val Ala Ser Gly Gln Lys Arg Glu Met
145                 150                 155                 160

Gly Ala Ser Leu Tyr Val Gly Trp Ala Ala Ser Gly Leu Leu Leu Leu
                165                 170                 175

Gly Gly Gly Leu Leu Cys Cys Asn Cys Pro Pro Arg Thr Asp Lys Pro
            180                 185                 190

Tyr Ser Ala Lys Tyr Ser Ala Ala Arg Ser Ala Ala Ala Ser Asn Tyr
        195                 200                 205

Val

<210>  11
<211>  472
<212>  DNA
<213>  Homo sapiens

<400>  11
ttttttgtgg caataattaa tatatttata tggaaattgt ctcatgcctt ataagctcct      60

aaaataaaat caaggtcatt atgtgactaa tgatagcact aggaggaaaa gaaaatactg     120

aaagtaaaac agtcttcagc cttggtttca gctatctctt caaatcagca ttaagtattt     180

aatatcagat ataacatttt tggcttgtca gaccagtaaa tcatgttttt ccactggaca     240

aaggaagctg aagaaacaac tgttgaacaa actaaatgct gtgacatgaa gcatctgact     300

tctaagtctg agtatttagt taaaatgatt ggtgaacatt taactgggca aaaggaatgg     360

catccatcat ccaaacaggc tcacttcccc cagcccccaa aatgaacagt agttttagat     420

agcaaagaaa taaaaagttt ttttcttttg attgtttaga gccaactgtg aa             472

<210>  12
<211>  504
<212>  DNA
<213>  Homo sapiens

<220>
<221>  misc_feature
<222>  (468)..(468)
<223>  n is a, c, g, or t

<220>
<221>  misc_feature
<222>  (491)..(491)
<223>  n is a, c, g, or t

<400>  12
tttcaaactc ctggactcaa gaaatccacc cacctcagcc tcccaaagtg ctaggattac      60

aggcatgagc cactgcgccc agcccttata aatttttgta tagacattcc tttggttgga     120

agaatattta taggcaatac agtcaaagtt tcaaaatagc atcacacaaa acatgtttat     180

aaatgaacag gatgtaatgt acatagatga cattaagaaa atttgtatga aataatttag     240

tcatcatgaa atatttagtt gtcatataaa aacccactgt ttgagaatga tgctactctg     300

atctaatgaa tgtgaacatg tagatgtttt gtgtgtattt ttttaaatga aaactcaaaa     360

taagacaagt aatttgttga taaatatttt taaagataac tcagcatgtt tgtaaagcag     420

gataccattt tacctaaaag gttcattggt tccaatccac agctcatngg tagagcaagg     480

aaggggtgga nggatggaaa ggat 504

<210> 13
<211> 242
<212> DNA
<213> Homo sapiens

<400> 13
tttttcctgt gcacaggtcc catttattgt agaaaataat aataattaca gtgatgaata 60

gctcttctta aattacaaaa cagaaaccac aaagaaggaa gaggaaaaac cccaggactt 120

ccaagggtga agctgtcccc tcctccctgc caccctccca ggctcattag tgtccttgga 180

aggggcagag gactcagagg ggatcagtct ccaggggccc tgggctgaag cgggtgaggc 240

ag 242